(19) **Europäisches Patentamt**
**European Patent Office**
**Office européen des brevets**

(11) **EP 2 028 474 B1**

(12) **EUROPÄISCHE PATENTSCHRIFT**

(45) Veröffentlichungstag und Bekanntmachung des Hinweises auf die Patenterteilung:
**04.08.2010 Patentblatt 2010/31**

(51) Int Cl.:
***G01N 15/06*** *(2006.01)*    ***G01N 33/28*** *(2006.01)*

(21) Anmeldenummer: **07024193.0**

(22) Anmeldetag: **13.12.2007**

(54) **Verfahren und Vorrichtung zum Erfassen von Partikeln in einer strömenden Flüssigkeit**

Method and device for measuring particles in a fluid stream

Dispositif et procédé destinés à détecter des particules dans un liquide s'écoulant

(84) Benannte Vertragsstaaten:
**AT BE BG CH CY CZ DE DK EE ES FI FR GB GR HU IE IS IT LI LT LU LV MC MT NL PL PT RO SE SI SK TR**

(30) Priorität: **21.08.2007 DE 102007039434**

(43) Veröffentlichungstag der Anmeldung:
**25.02.2009 Patentblatt 2009/09**

(73) Patentinhaber: **Prüftechnik Dieter Busch AG 85737 Ismaning (DE)**

(72) Erfinder:
- **Hölzl, Roland**
  **81369 München (DE)**
- **Becker, Edwin**
  **48734 Reken (DE)**
- **Knöll, Thomas**
  **89231 Neu-Ulm (DE)**

(56) Entgegenhaltungen:
**WO-A-2006/007826    WO-A-2007/088015**
**US-A- 4 837 511    US-A- 5 315 243**

**Beschreibung**

Die vorliegende Erfindung betrifft ein Verfahren zum Erfassen von elektrisch leitenden Partikeln in einer in einem Rohr strömenden Flüssigkeit mittels Wirbelströmen.

In der DE 2 108 717 A1 ist ein solches Verfahren und eine solche Vorrichtung beschrieben, wobei zwei Induktionsspulen in zwei Zweigen einer Wechselstrombrückenschaltung angeordnet sind, deren andere beiden Zweige durch die Hälften einer weiteren Spule gebildet werden. Die Spulen werden in axialer Richtung von der Flüssigkeit durchströmt und können in Strömungsrichtung hintereinander angeordnet sein, wobei die durch den Durchgang der Partikel hervorgerufenen Impedanzänderungen, bzw. der Unterschied der Impedanzänderung in beiden Spulen, ausgewertet wird. Es ist eine Anordnung gezeigt, bei welcher die Flüssigkeitsströmung in zwei parallel Teilstrecken aufgeteilt wird, die jeweils durch eine der beiden Spulen strömen, wobei dann ein axialer Versatz der Spulen nicht erforderlich ist.

Eine ähnliche Vorrichtung ist in der DE 28 40 358 A1 beschrieben.

Von der Firma momac GmbH & Co. KG, 47408 Moers, Deutschland, wird unter der Bezeichnung "metalscan" ein Gerät vertrieben, bei dem drei Spulen in Strömungsrichtung hintereinander angeordnet sind, wobei die erste und die letzte Spule als Senderspulen und die mittlere Spule als Empfängerspule wirken, um den Durchgang von elektrisch leitenden Partikeln aus einem Schmiermittelkreislauf zu erfassen. Die erste und die letzte Spule sind umgekehrt gepolt.

Weitere Vorrichtungen, bei denen das Signal von von der Flüssigkeit durchströmten Induktionsspulen zur Partikelerfassung verwendet wird, sind beispielsweise in der WO 2004/081608, WO 2004/104561, EP 0 778 937 A2 und EP 0 451 209 B1 beschrieben.

Aus der DE 39 31 497 A1 ist ein Verfahren zum induktiven Erfassen von Partikeln in Schmierstoffen bekannt, wobei eine in einer axial durchströmten Spule eingebettete Ankoppelspule resonant angeregt wird und der Durchgang von Partikeln anhand der dem Spulensystem durch die Wirbelströme entzogenen Energie erfasst wird. Dabei wird aus der Signalamplitude die Partikelgrösse bestimmt. Um eine Verfälschung der Messung durch bei Teilchendurchgang in der Spulenmitte im Vergleich zum Spulenrand abnehmende Empfindlichkeit der Spule zu vermeiden, wird durch einen Drallerzeuger dafür gesorgt, dass die Partikel die Spule immer nahe der Spulenwand passieren.

Aus der DE 31 17 319 A1 ist die Erfassung der Fließgeschwindigkeit von flüssigem Metall mittels Wirbelstrommessung unter Verwendung ein Kreuzkorrelationsfunktion beschrieben.

In der DE 40 14 756 A1 ist die Bestimmung der Geschwindigkeit eines Körpers oder Materials mittels Wirbelstrommessung beschrieben, wobei eine Korrelationsfunktion gebildet wird.

In der US 3,575,050 und der DE 28 50 246 A1 wird erwähnt, dass es Strömungsmessgeräte auf Wirbelstrombasis gibt.

Ein übliches Messverfahren zur zerstörungs- und berührungsfreien Erfassung von Fehlern in einem Prüfling, insbesondere metallischem Halbzeug, ist das Induzieren und Messen von Wirbelströmen in dem Prüfling. Dabei wird der Prüfling mittels einer sinusförmig bestromten Sendespule mit periodischen elektromagnetischen Wechselfeldern beaufschlagt. Die dadurch in dem Prüfling induzierten Wirbelströme induzieren wiederum in einer als Sonde verwendeten Spulenanordnung, die eine einzelne Spule ("Absolutspule") oder zwei subtraktiv geschaltete Spulen ("Differenzspule") aufweisen kann, ein periodisches elektrisches Signal, das eine Trägerschwingung entsprechend der Senderträgerfrequenz aufweist, deren Amplitude und/oder Phase durch einen Fehler in dem Prüfling in charakteristischer Weise moduliert wird, wenn ein Fehler in den empfindlichen Bereich der Sonde, d.h. in die Wirkbreite der Sonde, gelangt. Die Senderspule bildet dabei die Primärseite und die Empfängerspule(n) die Sekundärseite einer transformatorischen Anordnung. Ein Beispiel für eine solche Anordnung ist in der EP 1 189 058 A2 zu finden. Falls mehr als zwei Empfängerspulen verwendet werden, wird eine solche Anordnung auch als " Multi-Differenzspule" bezeichnet.

Üblicherweise wird zum Abtasten des Prüflings der Prüfling linear bezüglich der Sonde bewegt, wobei jedoch auch Anordnungen mit rotierender Sonde bekannt sind. Das von der Sonde erfasste Signal wird üblicherweise analog demoduliert, z.B. mittels Synchrondemodulation, und anschließend einer Auswertung unterzogen, um Fehler in dem Prüfling zu erkennen. Eine Digitalisierung des Signals erfolgt dabei üblicherweise erst für die Auswertung und Darstellung des Fehlersignals, d.h. nach der Demodulation des Spulensignals. Ein Beispiel für ein solches Verfahren ist in der DE 40 03 330 A1 zu finden.

In ähnlicher Weise verursachen elektrisch leitende Partikel in einer Flüssigkeit, welche die Spulen durchströmt, Wirbelstromverluste, die sich wiederum in einer meßbaren Impedanzänderung der Spulen niederschlagen. Auf diese Weise können mittels einer induktiven Spulenanordnung elektrisch leitende Partikel in einer in einem Rohr strömenden Flüssigkeit erfaßt werden. Dies ist besonders vorteilhaft, wenn man die Konzentration metallischer Partikel in einem Schmiermittelkreislauf einer Maschine erfassen will, um auf den Maschinenzustand rückzuschließen (die Konzentration der metallischen Partikel ist in der Regel ein Maß für den Verschleiß der Maschine)

Solche Wirbelstrommessverfahren sind aufgrund des für die analoge Demodulation erforderlichen Aufwands relativ aufwendig und kostspielig. Ferner ist zu berücksichtigen, dass üblicherweise für unterschiedliche Relativ-Geschwindigkeiten des Prüflings zur Sonde, d.h. bei unterschiedlichen Ausstoss- bzw. Testgeschwindigkeiten, unterschiedliche Filtersätze für das demodulierte Signal erforderlich sind, was bei variabler Prüflingsgeschwindigkeit einen zusätzlichen Aufwand mit sich bringt.

In der US 5,175,498 ist ein Wirbelstrommessverfahren beschrieben, bei welchem bereits das von der Spulensonde aufgenommene Messsignal mittels eines triggerbaren A/D-Wandlers digitalisiert und anschließend in digitaler Form einer Demodulation mittels Fouriertransformation unterzogen wird. Das Triggern des A/D-Wandlers, d.h. die Abtastrate, wird in Abhängigkeit von der mittels eines Encoders erfaßten Vorschubgeschwindigkeit des Prüflings gesteuert, um von einer Rückwärtsbewegung des Prüflings resultierende Fehler bei der Signalauswertung zu vermeiden.

In der US 4,445,088 ist ein Magnetstreufluss-Messverfahren beschrieben, in welchem ein metallischer Prüfling relativ zu einer Sonde bewegt wird, wobei das von der Sonde erfasste Messsignal nach Durchlaufen eines Bandpassfilters mittels eines triggerbaren A/D-Wandlers digitalisiert wird, wobei die Triggerung des Wandlers, d.h. die Abtastrate, von der mittels eines Geschwindigkeitssensors erfaßten Vorschubgeschwindigkeit des Prüflings gesteuert wird. Zur Fehlererkennung wird die Amplitude des digitalisierten Signals hinsichtlich der Überschreitung eines Schwellwerts ausgewertet, wobei die Wahl der Abtastrate in Abhängigkeit von der Prüfungsgeschwindigkeit dazu dient, eine von der Prüflingsgeschwindigkeit unabhängige vorgegebene Messgenauigkeit zu erzielen.

In der WO 2006/007826 A1 ist Wirbelstromessverfahren zur Erfassung von Fehlern in einem relativ zu einer durch eine Wirkbreite charakterisierte Sonde mit einer Geschwindigkeit (v) bewegten Prüfling beschrieben, wobei mittels der Sonde ein periodisches elektrisches Signal erfasst wird, das eine Trägerschwingung aufweist, deren Amplitude und/ oder Phase durch einen Fehler in dem Prüfling moduliert wird, wobei das Sondensignal gefiltert wird und mittels einer triggerbaren A/D-Wandlerstufe abgetastet wird, um ein demoduliertes digitales Messsignal zu erhalten, welches mittels einer digitalen frequenzselektiven, einstellbaren zweiten Filtereinheit gefiltert wird, um ein Nutzsignal zu gewinnen, welches ausgewertet wird, um einen Fehler in dem Prüfling zu erkennen, wobei die A/D-Wandlerstufe mit einem $n$-ten, ganzzahligen Bruchteil der Frequenz der Trägerschwingung getriggert wird, wobei $n$ in Abhängigkeit von der Fehlerfrequenz, die sich als Quotient aus der Relativ-Geschwindigkeit zwischen Prüfling und Sonde und der Wirkbreite der Sonde ergibt, gewählt wird, und wobei die frequenzselektive zweite Filtereinheit in Abhängigkeit von der Fehlerfrequenz eingestellt wird.

Es ist Aufgabe der vorliegenden Erfindung, ein besonders einfaches Verfahren zum Erfassen von elektrisch leitenden Partikeln in einer in einem Rohrstück strömenden Flüssigkeit zu schaffen.

Diese Aufgabe wird erfindungsgemäß gelöst durch ein Verfahren gemäß Anspruch 1.

Ein wesentlicher Aspekt der vorliegenden Erfindung besteht darin, dass das Sondensignal mit einem ganzzahligen Bruchteil der Frequenz der Trägerschwingung abgetastet, d.h. digitalisiert, wird, d.h. es findet eine Unterabtastung der Trägerschwingung statt.

Auf diese Weise wird die Trägerschwingung aus dem Messsignal eliminiert, wodurch die ansonsten übliche Demodulation des Messsignals entfällt und somit eine wesentliche Vereinfachung des Verfahrens und eine wesentliche Verringerung des Aufwands für eine ansonsten erforderliche Demodulation, z.B. analoge Synchrondemodulation, erzielt werden kann, was zu erheblichen Kosteneinsparungen und gegebenenfalls auch zu Bauraumeinsparungen führt.

Ferner ermöglicht die Unterabtastung die Verwendung von A/D-Wandlern mit sehr hoher Auflösung, die üblicherweise relativ langsam, d.h. in ihrer maximalen Abtastrate relativ beschränkt, sind.

Außerdem führt die Unterabtastung dazu, dass das Nutzsignal mit einer relativ niedrigen Datenrate erhalten wird, was wiederum die Darstellung des Nutzsignals erleichtert, so dass beispielsweise Standardbussysteme und eventuell Funkbussysteme verwendet werden können, was bei hohen Datenraten nicht oder nur nach Datenkompression möglich wäre.

Auch erlaubt die Unterabtastung das Ausführen des Messverfahrens mit relativ niedrigem Energieverbrauch, wobei sogar während Zeitintervallen, in welchen keine Abtastung erfolgt, der Sender abgeschaltet werden könnte, falls beispielsweise nur bei jeder zehnten Periode der Trägerschwingung oder noch seltener abgetastet wird. Dieser Aspekt ist insbesondere für tragbare Geräte im Batteriebetrieb von Bedeutung oder falls eine kabellose, d.h. drahtlos mit der Auswerteeinheit verbundene, Sonde verwendet werden soll.

Schließlich kann sich bei der Unterabtastung eine im Vergleich zu quasi-kontinuierlicher Abtastung verringerte Anfälligkeit gegenüber nichtperiodischen Störsignalen ergeben, da solche Störsignale, sofern sie nicht in den jeweiligen Abtastzeitraum fallen, im Nutzsignal überhaupt nicht wahrgenommen werden, während sich bei quasi-kontinuierlicher Abtastung alle Störsignale im Nutzsignal niederschlagen.

Vorzugsweise umgibt die Senderspule das Rohrstück, wobei die Spulenanordnung mindestens eine erste das Rohrstück umgebende induktive Empfängerspule und eine zweite das Rohrstück umgebende, axial zur ersten Empfängerspule beabstandete, induktive Empfängerspule aufweist, die im Bereich der Senderspule angeordnet sind und subtraktiv geschaltet sind, um ein Differenzsignal entsprechend den von der Senderspule induzierten Wirbelströmen auszugeben, wobei die Senderspule die Primärseite und die Empfängerspulen die Sekundärseite einer transformatorischen Anordnung bilden, und wobei das Sondensignal von dem Differenzsignal gebildet wird. Auf diese Weise sind alle Spulen nahe beieinander angeordnet und damit im wesentlichen den gleichen Umwelteinflüssen, z.B. hinsichtlich Temperatur ausgesetzt, was die Meßgenauigkeit erhöht.

Gemäß einem weiteren wesentlichen Aspekt der Erfindung wird die Abtastrate, d.h. der Grad der Unterabtastung, in Abhängigkeit von einer Partikelfrequenz, die als Quotient aus der Störmugsgeschwindigkeit der Flüssigkeit und der Wirkbreite der Sonde definiert ist, gewählt.

Da die Dauer des von einem Partikel in der Flüssigkeit verursachten Nutzsignals, und damit die Partikelfrequenz, im wesentlichen nur von der Ausdehnung des empfindlichen Bereichs der Sonde, d.h. der Wirkbreite, und der Strömungsgeschwindigkeit der Flüssigkeit abhängt, kann auf diese Weise einerseits sichergestellt werden, dass die Genauigkeit der Darstellung des Nutzsignals nicht von der Strömungsgeschwindigkeit abhängt (durch entsprechende Wahl der Abtastrate kann sichergestellt werden, dass immer in etwa die gleiche Anzahl von Abstastpunkten in jedes Partikelsignal fällt), und andererseits kann sichergestellt werden, dass das Signal eines Partikels unabhängig von der Strömungsgeschwindigkeit im wesentlichen gleich aussieht, was das Erkennen des Partikeldurchgangs erleichtert.

Ein weiterer Vorteil der an die Strömungsgeschwindigkeit angepassten Unterabtastung besteht darin, dass auf diese Weise die digitale Filtereinheit, mittels welcher das von der A/D-Wandlerstufe gelieferte digitale Messsignal gefiltert wird, um ein störungsfreies Nutzsignal zu gewinnen, auf sehr einfache Weise in Abhängigkeit von der Partikelfrequenz eingestellt werden kann, nämlich durch Takten des digitalen Filters mit der Abtastrate (bei einem digitalen Filter hängt die Eckfrequenz direkt von der Taktrate ab). Auf diese Weise kann mit einem einzigen Filtersatz gearbeitet werden, der durch entsprechende Wahl der Abtastrate, d.h. Taktrate, automatisch mit seinen Eckfrequenzen an die von der Strömungsgeschwindigkeit abhängigen Bandbreite des Partikelsignals angepasst ist.

Die Partikelfrequenz entspricht üblicherweise dem Maximum des Partikelspektrums, d.h. der Frequenz mit der höchsten Intensität. Die Bandbreite des Partikelsignals ist der Frequenzbereich um die Partikelfrequenz herum, in welchem noch für die Partikelerkennung maßgebliche Informationen zu finden sind. Die Wirkbreite der Sonde hängt einerseits von der geometrischen Ausgestaltung der Sonde ab, jedoch auch von den Rahmenbedingungen des Einsatzes der Sonde ab. Physikalisch entspricht die Wirkbreite der Länge, die der reziproken Partikelfrequenz entspricht und sich aus der Strömungsgeschwindigkeit dividiert durch das Zweifache der Partikelfrequenz ergibt. Die Wirkbreite gibt somit die Länge an, über welche ein Partikel das von der Sonde aufgenommene Messsignal beeinflussen kann.

Die Wechselspannung für die Senderspule kann aus einem binären Signal durch Kurvenformung erzeugt werden, wobei vorzugsweise das Triggersignal für die A/D-Wandlerstufe dadurch erzeugt wird, dass die Frequenz des für die Erzeugung der Senderwechselspannung verwendeten binären Signals durch eine ganze Zahl geteilt wird. Die ganze Zahl, durch welche die Frequenz der Trägerschwingung geteilt wird, um die A/D-Wandlerstufe zu triggern, wird vorzugsweise umgekehrt proportional zu der Partikelfrequenz gewählt, so dass die gewählte Abtastrate mindestens annähernd proportional zur Partikelfrequenz gewählt werden kann. Vorzugsweise wird diese ganze Zahl so gewählt, dass in ein Basisintervall, d.h. ein Zeitintervall, das dem Inversen der Partikelfrequenz entspricht, mindestens 5, vorzugsweise mindestens 20, jedoch höchstens 100, vorzugsweise höchstens 50, Abtastungen durch die A/D-Wandlerstufe fallen.

Wie bereits erwähnt, kann das Einstellen der frequenzselektiven zweiten Filtereinheit in Abhängigkeit von der Partikelfrequenz selbsttätig dadurch erfolgen, dass die zweite Filtereinheit mit der Abtastrate per A/D-Wandlerstufe getaktet wird, da bei einem digitalen Filter die Eckfrequenz direkt proportional zur Taktfrequenz ist. Zweckmäßigerweise weist die zweite Filtereinheit einen Tiefpass auf, um Anteile oberhalb der Partikelbandbreite auszublenden, sowie einen Hochpass, um Gleichanteile des digitalen Signals anzublenden. Zweckmäßigerweise liegt die Eckfrequenz des Tiefpasses über der Partikelfrequenz, vorzugsweise über dem Zweifachen der Partikelfrequenz, während die Eckfrequenz des Hochpasses unter der Partikelfrequenz, vorzugsweise unter einem Viertel der Partikelfrequenz liegt. Da die bevorzugten Eckfrequenzen der zweiten Filtereinheit direkt von der Basisfrequenz abhängen, kann dadurch, dass die Abtastrate des Signals ebenfalls in Abhängigkeit von der Partikelfrequenz gewählt wird und die zweite Filtereinheit mit dieser Abtastrate getaktet wird, auf besonders einfache Weise für eine automatische optimale Anpassung der Eckfrequenzen der Filtereinheit an die Partikelfrequenz, d.h. die Strömungsgeschwindigkeit und die Wirkbreite der Sonde, erzielt werden. Grundsätzlich können die Eckfrequenzen näher an der Partikelfrequenz liegen als bei herkömmlichen Verfahren, da durch die genaue Nachführung der Filter hinsichtlich der Partikelfrequenz, d.h. insbesondere der Strömungsgeschwindigkeit, die Gefahr, dass die Filter für die Erfassung eines Partikeldurchgangs nutzbare Information abschneiden, verringert ist.

Vorzugsweise wird die Frequenz der Trägerschwingung so gewählt, dass sie mindestens das zehnfache, noch besser mindestens das zwanzigfache, der Partikelfrequenz beträgt, da ansonsten die Reproduzierbarkeit einer Partikelerfassung (d.h. die Reproduzierbarkeit des für einen Partikeldurchgangs typischen Signals) beeinträchtigt sein kann, was die Partikelerfassung erschweren würde.

Obschon grundsätzlich auch Lösungen möglich sind, bei welchen nur ein einzelner Wert pro abgetasteter Vollwelle erfasst wird, wobei dann die Phasenlage auf indirekte Weise bestimmt werden müsste, werden vorzugsweise pro abgetasteter Vollwelle zwei Werte mit einem festen Phasenversatz gewonnen, wobei dies vorzugsweise mit zwei A/D-Wandlern durchgeführt wird, d.h. die Wandlerstufe weist zwei parallel geschaltete A/D-Wandler auf, die mit gleicher Frequenz so getriggert werden, dass sie um eine feste Phasendifferenz versetzt abtasten, wobei die Phasendifferenz vorzugsweise 90° oder ein ganzteiliges Vielfaches von 360° plus 90° beträgt (die Phasendifferenz muss aber nicht unbedingt genau 90° betragen, sondern könnte beispielsweise zwischen 85 und 95° liegen). Mittels einer solchen phasenversetzten Abtastung kann sichergestellt werden, dass trotz der Unterabtastung die maximale Signalinformation gewonnen wird, und es wird das digitale Messsignal als zweikomponentiges Signal, d.h. mit Phasen- und Amplitudeninformation, erhalten, wodurch die Partikelerfassung verbessert wird. Dabei ist es zweckmäßig, dass die beiden Komponenten des von der mit zwei A/D-Wandlern ausgestatteten A/D-Wandlerstufe gelieferten digitalen Messsignals separat mittels der zweiten Filtereinheit gefiltert werden, um das Nutzsignal als zweikomponentiges Signal zu gewinnen, wobei dann bei der Auswertung des Nutzsignals beide Komponenten berücksichtigt werden können.

Um eine solche phasenversetzte Abtastung durchzuführen, ist es nicht notwendigerweise erforderlich, genau zwei A/D-Wandler zu verwenden. Statt dessen könnte auch nur ein einziger, hinreichend schneller A/D-Wandler verwendet werden, der beide Abtastungen, d.h. z.B. diejenige bei 0° und diejenige bei 90°, vornimmt, wobei diese beiden Abtastwerte dann wie bei der Verwendung von zwei A/D-Wandlern getrennt weiterverarbeitet werden, um eine zweikomponentige Signalauswertung zu erreichen. Eine Verwendung von sehr langsamen A/D-Wandlern wäre möglich, wenn z.B. 4, 8, oder 16, usw. A/D-Wandler verwendet werden, die dann nicht bei jeden Triggerimpuls an die A/D-Wandlerstufe aktiv werden, sondern nur bei jedem 2, 4, bzw. 8-ten, usw., d.h. die Abtastungsarbeit für jede der beiden Phasenlagen wird entsprechend zeitlich auf jeweils mehrere A/D-Wandler aufgeteilt.

Vorzugsweise weist die A/D-Wandlerstufe eine Auflösung von mindestens 16 Bit auf, wobei vorzugsweise Flash-Wandler oder SAR-Wandler verwendet werden.

Grundsätzlich kann die Beaufschlagung der Flüssigkeit mittels der Senderspule, d.h. die Einstrahlung der elektromagnetischen Wechselfelder, mindestens für einen Teil eines jeden Intervalls zwischen zwei aufeinander folgenden Triggersignalen für die A/D-Wandlerstufe unterbrochen werden, da in dieser Zeit ohnehin keine Signalerfassung, d.h. Abtastung, stattfindet (je nach Einzelfall können diese Abtastpausen unter Umständen sich über viele Perioden der Trägerschwingung erstrecken). Auf diese Weise kann insbesondere bei tragbaren Messgeräten eine erhebliche Einsparung des Energieverbrauchs erzielt werden, wodurch beispielsweise die Stromversorgungselemente in Abmessungen und Gewicht wesentlich reduziert werden können. In ähnlicher Weise kann auch auf der Signalerfassungsseite die Elektronik, d.h. insbesondere der Signalverarbeitungsprozessor, zur Energieersparnis während des Intervalls zwischen zwei Abtastungen stillgelegt werden.

Die erste Filtereinheit weist vorzugsweise einen Tiefpaß auf, der als Aliasing-Filter bezüglich der Abtastung durch die A/D-Wandlerstufe wirkt, wobei ferner vorzugsweise ein Hochpaß vorgesehen ist, um niederfrequente Störsignale auszublenden. Die erste Filtereinheit wird üblicherweise als Analogfilter ausgebildet sein. Ferner ist vorzugsweise nach der ersten Filtereinheit ein steuerbarer Verstärker vorgesehen, um das Filtersignal auf die für die A/D-Wandlerstufe optimal geeignete Amplitude zu bringen.

Die Steuerung der A/D-Wandlerstufe, d.h. die Wahl der Triggerzeitpunkte, sowie die Verarbeitung des von der A/D-Wandlerstufe gelieferten digitalen Signals erfolgt vorzugsweise mittels eines digitalen Signalprozessors, der vorzugsweise auch die zweite Filtereinheit bildet. Die Ansteuereinrichtung zum Triggern der A/D-Wandlerstufe weist vorzugsweise eine Quelle für ein binäres Signal auf, die von einem Timer gebildet werden kann, und einen Teiler auf, mittels welchem das binäre Signal durch eine ganze Zahl geteilt wird, um das Triggersignal für die A/D-Wandlerstufe zu erzeugen, wobei das binäre Signal von einem Kurvenformer bearbeitet wird, um die Versorgungsspannung für den Sender zu liefern. Der Timer kann als Teil des digitalen Signalsprozessors oder separat davon ausgebildet sein. Der Teiler ist vorzugsweise separat von dem Signalprozessor als PAL (Programmable Array Logic)-Baustein ausgebildet.

Grundsätzlich kann die Strömungsgeschwindigkeit der Flüssigkeit durch Messung bestimmt werden. Typischerweise ist das Rohrstück Teil eines Schmiermittel-Kreislaufs, wobei es sich bei der Flüssigkeit um Schmieröl einer Maschine handelt. In solchen Fällen ist der Flüssigkeitsdurchsatz üblicherweise bekannt und im wesentlichen konstant, so dass dann keine Messung Strömungsgeschwindigkeit der Flüssigkeit erforderlich ist und die Geschwindigkeit als Parameter für die Signalerfassung bzw. -auswertung fest vorgegeben werden kann.

Im folgenden wird die Erfindung anhand der beigefügten Zeichnungen beispielhaft näher erläutert. Dabei zeigen:

Fig. 1 schematisch den Aufbau eines Ausführungsbeispiels einer Wirbelstrom- Messvorrichtung zum Erfassen von leitenden Partikeln in einer Flüssigkeit

Fig. 2 schematisch einen beispielhaften Verlauf des Sondensignals bei der digitalen Abtastung;

Fig. 3 in schematischer Weise einen Längsschnitt durch ein von einer Flüssigkeit durchströmtes Rohr, welches mit Sender- bzw. Sondenspulen zur Verwendung mit der Messvorrichtung von Fig. 1 versehen ist;

Fig. 4 ein Blockschaltbild der Verschaltung der Spulen von Fig. 3;

Fig. 5 einen idealisierten Verlauf des Betrags des Signals der Empfängerspulen von Fig. 3, wobei die Messwerte unterhalb eines Schwellwerts abgeschnitten wurden;

Fig. 6    eine schematische Darstellung der theoretischen radialen Geschwindigkeitsverteilung in einer laminaren Strömung in einem Rohr;

Fig. 7    ein Beispiel für die Abhängigkeit der Wirkbreite der Empfängerspulen von Fig. 3 von der radialen Position eines erfassten Partikels; und

Fig. 8    ein Beispiel für die Abhängigkeit der Dämpfung des Signals der Empfängerspulen von Fig. 3 von der radialen Position eines erfassten Partikels.

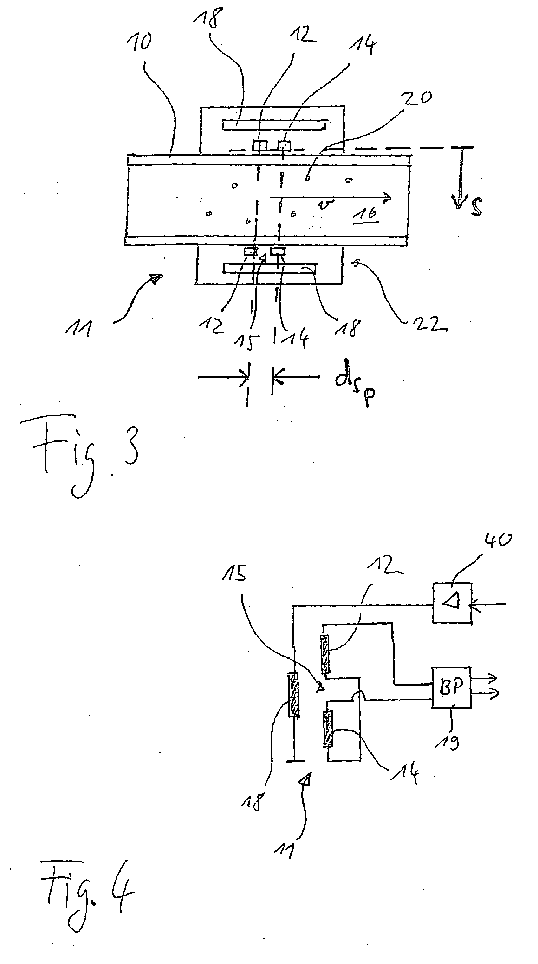

Gemäß Fig. 3 ist ein Rohrstück 10 von einer ersten induktiven Empfängerspule 12 und einer davon in axialer Richtung beabstandet angeordneten zweiten induktiven Empfängerspule 14 umgeben, so dass eine in dem Rohrstück 10 strömende Flüssigkeit 16 die Spulen 12 und 14 in axialer Richtung durchströmt. Der axiale Abstand der beiden Spulen 12, 14 und die axiale Abmessung der Spulen 12, 14 kann beispielsweise 2 mm betragen. Die beiden Empfängerspulen 12, 14 sind außen von einer Senderspule 18 umgeben, die koaxial zu den beiden Spulen 12, 14 angeordnet ist und einen größeren Durchmesser als diese aufweist. Die axiale Abmessung der Senderspule 18 ist dergestalt, dass die beiden Empfängerspulen 12, 14 vollständig innerhalb der Senderspule 18 angeordnet sind. Vorzugsweise ist die Ausdehnung der Senderspule 18 in axialer Richtung mindestens doppelt so groß ist wie die axiale Ausdehnung der Anordnung der Empfängerspulen 12, 14, d.h. Abstand plus axiale Ausdehung der Spulen 12, 14. Die Spulen 12, 14, 18 sind in einem das Rohrstück 10 umschließenden Gehäuse 22 angeordnet und bilden einen Meßkopf 11.

Typischerweise ist das Rohrstück 10 Teil eines Schmiermittelkreislaufs einer Maschine, wobei es sich bei der Flüssigkeit 16 dann um ein Schmiermittel handelt, in welchem metallische Partikel vorhanden sind, bei denen es sich typischerweise um Abrieb aus bewegten Teilen der Maschine handelt. Ein typischer Wert für den Schmiermitteldurchsatz im Hauptstrom ist 10 Liter/min. Bei wesentlich höheren Durchsätzen ist es zweckmäßig, nicht im Hauptstrom, sondern in einem Nebenstrom zu messen.

Gemäß Fig. 4 sind die beiden Empfängerspulen 12, 14 subtraktiv als Differenzspule 15 geschaltet, d.h. sind gegenläufig angeordnet, so dass in den beiden Spulen 12, 14 eine Spannung mit gleichem Betrag aber entgegengesetzten Vorzeichen induziert wird. Insgesamt bilden die Senderspule 18 und die Empfängerspulen 12, 14 eine transformatorische Anordnung, wobei die Senderspule 18 die Primärseite bildet und die Empfängerspulen 12, 14 die Sekundärseite bilden. Der Transformatorkern wird bei einer solchen Anordnung von den die Spulen 12, 14, 18 durchsetzenden Materialien bzw. Medien, d.h. Luft, dem Gehäuse 22, dem Rohr 10, sowie der Flüssigkeit 16 mit den Partikeln 20, gebildet.

Der durch die Partikel 20 verursachte Impedanzunterschied der Spulen 12, 14, d.h. der durch die momentane Anwesenheit eines Partikels 20 in einer der beiden Spulen 12, 14 (die Partikel 20 sind wesentlich kleiner als der Abstand der Spulen 12, 14) verursachte Unterschied der Impedanz der beiden Spulen 12, 14 wird durch das von den Spulen 12 und 14 ausgegebene Meßsignal abgebildet.

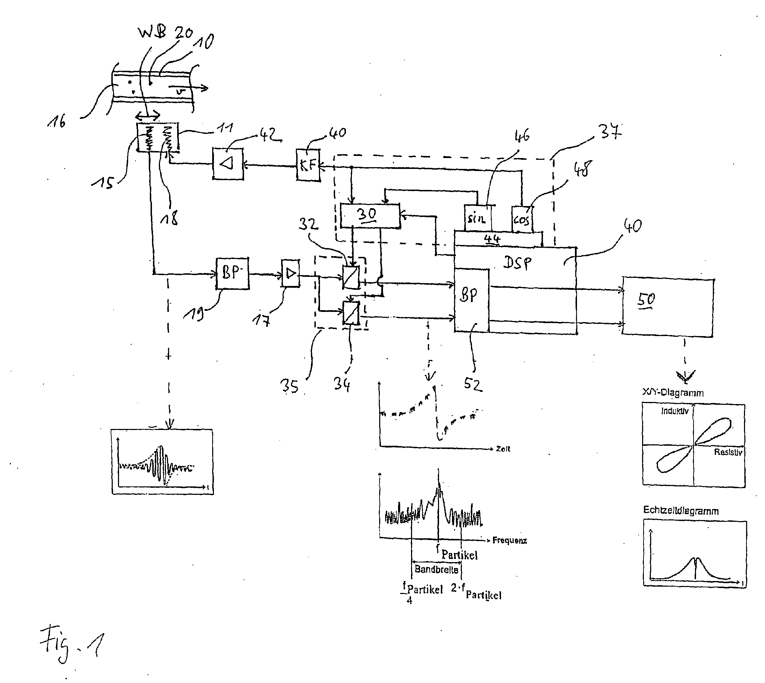

In Fig. 1 ist ein Beispiel für den Aufbau einer Wirbelstrommessvorrichtung gezeigt, welche den Meßkopf 11 verwendet.

Die Sendespule 18 dient dazu, mittels eines elektromagnetischen Wechselfeldes mit mindestens einer vorgegebenen Trägerfrequenz Wirbelströme in den Partikeln 20 zu induzieren, die in der als Differenzspule ausgebildeten Empfangsspule 15 wiederum eine als Sondensignal wirkende Wechselspannung induzieren, die eine Trägerschwingung mit der Trägerfrequenz der Sendespule 18 aufweist, wobei die Amplitude und die Phase des Sondensignals durch ein Partikel 20 moduliert wird, wenn dieses in die Wirkbreite WB der Empfangsspule 15 gelangt.

Die Spannung für die Sendespule 18 kann beispielsweise dadurch erzeugt werden, dass ein von einer Timer-Einheit 44 erzeugtes binäres Signal an einen Generator 48 als Vorgabefrequenz geliefert wird, der daraus ein Rechtecksignal oder auch ein Sinussignal erzeugt, welches einen Kurvenformer 40 durchläuft und anschließend mittels eines Leistungsverstärkers 42 verstärkt wird, bevor es der Sendespule 18 zugeführt wird. Vorzugsweise hat das Signal sinusartige Form und enthält im einfachsten Fall nur eine einzige Trägerfrequenz, wobei jedoch grundsätzlich auch Messungen mit gleichzeitig mehreren Trägerfrequenzen und/oder von Sinus-Schwingungen deutlich abweichenden Trägersignalen möglich. Vorzugsweise liegt die Trägerfrequenz im Bereich von 20 kHz bis 500 kHz.

Das von der Empfangsspule 15 aufgenommene Sondensignal durchläuft einen Bandpaß 19 sowie eine einstellbaren Vorverstärker 17, bevor es einer A/D-Wandlerstufe 35 zugeführt wird. Der Bandpass 19 dient einerseits mittels des Tiefpasses als (Anti-)Aliasing-Filter hinsichtlich der Digitalisierung des Signals durch die A/D-Wandlerstufe 35 sowie andererseits mittels des Hochpasses zum Ausblenden von niederfrequenten Störsignalen. Der einstellbare Vorverstärker 17 dient dazu, die Amplitude des analogen Sondensignals auf die für die A/D-Wandlerstufe 35 optimal geeignete Amplitude zu bringen.

Die A/D-Wandlerstufe 35 weist zwei parallel geschaltete A/D-Wandler 32 und 34 auf, welche eine hohe Auflösung, jedoch mindestens eine Auflösung von 16 Bit, vorzugsweise mindestens 22 Bit, haben sollten und vorzugsweise mindestens 500 A/D-Wandlungen pro Sekunde durchführen können sollten. Die A/D-Wandler 32, 34 sind vorzugsweise als Flash-Wandler oder SAR (Sukzessives Approximations-Register)-Wandler ausgebildet.

Die A/D-Wandlerstufe 35 wird von einer Ansteuereinrichtung 37 getriggert, welche die bereits erwähnte Timer-Einheit 44, den Cosinus-Generator 48, einen parallel dazu angeordneten Sinus-Generator 46 sowie einen Frequenzteiler 30 aufweist. Eingangsseitig liegt an dem Frequenzteiler 30 das von dem Cosinus-Generator 48 erzeugte Signal, welches die Frequenz der Trägerfrequenz des Versorgungssignals der Sendespule 18 hat, sowie das Signal des Sinus-Generators 46 an, welches dem Signal des Cosinus-Generators 48 entspricht, jedoch um 90° dazu phasenverschoben ist. In dem Frequenzteiler 30 werden diese beiden Signale hinsichtlich ihrer Frequenz durch eine ganze Zahl $n$ geteilt. Das entsprechende frequenzreduzierte Ausgangssignal dient dazu, den A/D-Wandler 32 bzw. den A/D-Wandler 34 zu triggern. Die Auswahl der Zahl $n$ für den Teiler 30 wird von einem digitalen Signalprozessor 40 in Abhängigkeit von einer "Partikelfrequenz", die als der Quotient aus der Strömungsgeschwindigkeit $v$ der Flüssigkeit 16, d.h. der Geschwindigkeit der Partikel 20, und der Wirkbreite WB der Empfangsspule 15, vorgenommen. Vorzugsweise wird $n$ umgekehrt proportional zu der Partikelfrequenz gewählt, um zu erreichen, dass die Triggerrate der A/D-Wandlerstufe 35 mindestens annähernd proportional zu der Partikelfrequenz ist. Auf diese Weise wird erreicht, dass, wenn man die Wirkbreite WB in erster Näherung als konstant annimmt, bei höherer Strömungs-/Partikelgeschwindigkeit $v$ und damit höherer Partikelfrequenz das analoge Sondensignal entsprechend häufiger abgetastet wird.

Vorzugsweise ist der Teiler 30 als sogenanntes PAL (Programmable Array Logic)-Baustein ausgebildet, um sicher zu stellen, dass die Triggersignale möglichst unverzögert, d.h. synchron, zu dem Ausgangssignal des Cosinus-Generators 48 und Sinus-Generators 46 und ohne Phasenjitter bei der A/D-Wandlerstufe 35 ankommen.

Aufgrund der entsprechenden Phasenverschiebung der beiden Eingangssignale des Teilers 30 erfolgt auch die Triggerung der beiden A/D-Wandler 32, 34 mit einem festen Phasenversatz von 90°. Auf diese Weise kann das analoge Sondensignal zweikomponentig ausgewertet werden, d.h. sowohl hinsichtlich Amplitude als auch Phase. Es versteht sich, dass die Phasenverzögerung zwischen dem Triggersignal der A/D-Wandlerstufe 35 und dem Signal der Sendespule 18 möglichst gering sein sollte, wobei insbesondere auch ein sogenannter Phasenjitter vermieden werden sollte, d.h. die Phasenbeziehungen sollten zeitlich möglichst genau konstant sein.

Mit der gezeigten Ansteuereinrichtung 37 wird sichergestellt, dass das analoge Sondensignal von jedem A/D-Wandler 32 bzw. 34 höchstens einmal pro Vollwelle der Trägerschwingung abgetastet wird (in diesem Fall ist $n$ gleich 1). Je nach aktueller Partikelfrequenz, d.h. Partikelgeschwindigkeit $v$, kann $n$ jedoch wesentlich größer als 1 werden, so dass nur noch in jeder $n$-ten Vollwelle der Trägerschwingung überhaupt eine Abtastung erfolgt.

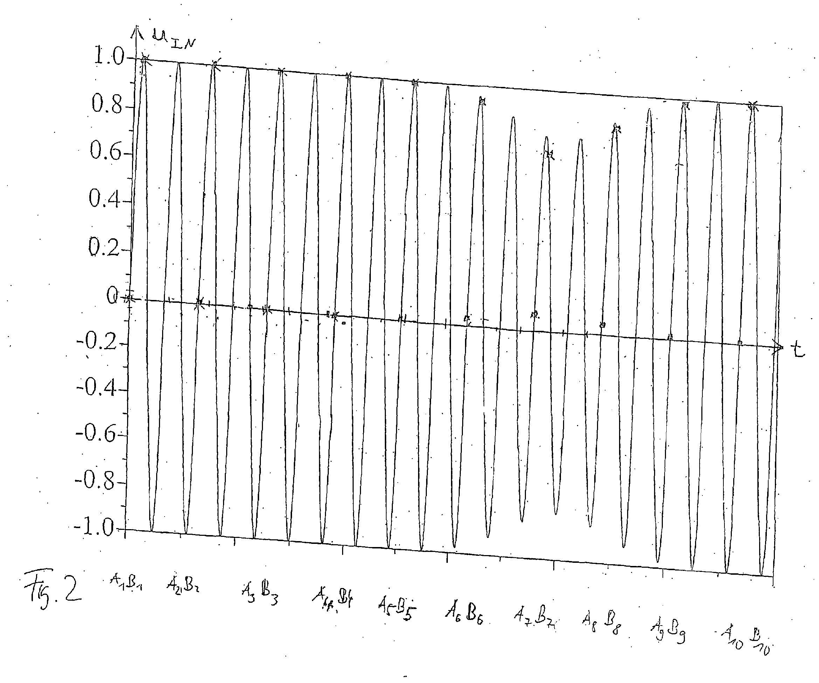

In Fig. 2 ist ein Beispiel gezeigt, in welchem $n$ gleich 2 ist, d.h. es wird nur in jeder zweite Vollwelle von jedem A/D-Wandler 32, 34 je eine Abtastung An bzw. $B_n$ vorgenommen.

Da in allen Fällen jedoch je A/D-Wandler 32, 34 höchstens einmal pro Vollwelle abgetastet wird, wird durch diese Unterabtastung die Frequenz der Trägerschwingung, d.h. die Trägerfrequenz, aus dem digitalen Signal eliminiert, d.h. mittels der Unterabtastung erfolgt eine Demodulation des analogen Sondensignals.

Vorzugsweise wird $n$ so gewählt, dass in dem Zeitintervall, in welchem ein nennenswertes Partikelsignal beobachtet wird, d.h. in dem Zeitintervall, in welchem sich ein Punkt eines Partikels 20 durch die Wirkbreite WB der Empfangsspule 15 bewegt, d.h. in dem Zeitintervall, das im wesentlichen dem Inversen der Partikelfrequenz entspricht, mindestens 5, vorzugsweise mindestens 20, Abtastungen durch jeden A/D-Wandler 32 bzw. 34 vorgenommen werden, um die in dem Partikelsignal enthaltene Information noch in einer für eine sichere Partikelerkennung ausreichenden Weise zu erhalten. In der Regel werden jedoch nicht mehr als 50, höchstens 100, Abtastungen während eines solchen Zeitintervalls erforderlich sein.

Die Frequenz der Trägerschwingung sollte so gewählt werden, dass sie mindestens das Zehnfache der Partikelfrequenz beträgt, da ansonsten das Partikelsignal von zu wenig Vollwellen der Trägerschwingung getragen wird und die Reproduzierbarkeit der Partikelerfassung problematisch wird..

Das demodulierte digitale zweikanalige Ausgangssignal der A/D-Wandlerstufe 35 durchläuft einen digitalen Bandpass 52, der von dem Signalprozessor 40 dargestellt werden kann und der dazu dient, Störsignale, die außerhalb der Bandbreite des Partikelsignals liegen, auszublenden. Zu diesem Zweck wird die Eckfrequenz des Hochpasses vorzugsweise so gewählt, dass sie weniger als ein Viertel der Partikelfrequenz beträgt, während die Eckfrequenz des Tiefpasses vorzugsweise so gewählt wird, dass sie mindestens das Zweifache der Partikelfrequenz beträgt, um das Ausblenden von Signalanteilen, die noch Information bezüglich des Partikeldurchgangs enthalten, zu vermeiden.

Der digitale Bandpass 52 wird mit der Abtastrate der A/D-Wandlerstufe 35, d.h. der Triggerrate, getaktet, was den großen Vorteil beinhaltet, dass die Eckfrequenzen des Bandpasses bei Änderung der Partikelfrequenz, d.h. bei Änderung der Partikelgeschwindigkeit $v$, automatisch mit der Partikelfrequenz mitgezogen werden, da die Eckfrequenzen eines digitalen Bandpasses proportional zur Taktrate sind und die Taktrate über die Abtastrate, welche von der Ansteuereinheit 37 vorgegeben wird, automatisch an die Änderung der Partikelfrequenz angepasst wird.

Die für die Bestimmung der Partikelfrequenz erforderliche Information bezüglich der Wirkbreite WB kann dem Signalprozessor 40 entweder manuell eingegeben werden oder sie wird direkt von dem Meßkopf 11 bereitgestellt, wie dies beispielsweise in der EP 0 734 522 B1 beschrieben ist.

Es versteht sich, dass das Messsystem analog auf eine Änderung der Partikelfrequenz reagiert, die dadurch verursacht ist, dass zwar die Partikelgeschwindigkeit $v$ konstant gehalten wird, jedoch die Empfangsspule 15 gegen eine andere mit unterschiedlicher Wirkbreite WB ausgetauscht wird.

Insbesondere bei relativ großen Werten von *n*, d.h. wenn nur eine relativ kleine Zahl der Vollwellen der Trägerschwingung überhaupt abgetastet wird, kann während der Abtastpausen beispielsweise die Sendespule 18 und/oder die Auswerteelektronik, d.h. insbesondere der Signalprozessor 40, ausgeschaltet bzw. ruhig gestellt werden, um eine Verringerung der Leistungsaufnahme zu erzielen, was insbesondere für tragbare Messgeräte von Bedeutung ist.

Das nach Filterung durch den digitalen Bandpass 52 erhaltene Nutzsignal wird in einer Auswerteeinheit 50 ausgewertet, um den Durchgang von Partikeln 20 zu erfassen, wobei hier üblicherweise sowohl die Amplituden- als auch Phaseninformation des Partikel signals herangezogen wird.

Zweckmäßigerweise ist die Auswerteeinheit 50 so ausgebildet, dass eine Zählung der erfaßten Partikeldurchgänge erfolgt, um auf die Partikelkonzentration in der Flüssigkeit 16, und damit gegebenenfalls auf den Maschinenzustand, rückschließen zu können.

Grundsätzlich ergibt sich bei einer Differenzspule als Folge der Differenzbildung (die einzelnen Spulen der Differenzspule sind in der Praxis nie genau gleich) die sog. Spulenfehlspannung, die das eigentliche Differenzsignal z.B. um bis zu drei Größenordnungen übersteigen kann. Die sich daraus ergebende relativ große Amplitude des Empfängerspulensignals im Vergleich zum eigentlichen Nutzsignal stellt hohe Anforderungen an den A/D-Wandler, insbesondere an dessen Auflösung.

Falls die zu einem akzeptablen Preis verfügbaren A/D-Wandler für das Empfängerspulensignal nicht diesen Anforderungen genügen, ist es möglich, die Spulenfehlspannung mit einem D/A-Wandler zu kompensieren, bevor sie dem A/D-Wandler zugeführt wird. Dabei wird vor der A/D-Wandlung das Empfängerspulensignal mit einem Offset beaufschlagt, der zuvor in einer MIttelwertbildung mit geringer Verstärkung aus dem (demodulierten) Empfängerspulensignal ermittelt wurde (in der komplexen Darstellung des Empfägerspulensignals wird mittel der Kompensation der Spulenfehlspannung durch Beaufschlagung mit einem geeigneten Offset mittels des DIA_Wandlers das Nutzsignal in den oder nahe an den Ursprung der komplexen Ebene gebracht; ein verbleibender Rest der Spulenfehlspannung kann bei der Verarbeitung des Ausgangssignals des A/D-Wandlers eliminiert werden). Während der Messung kann dieser Offsetwert weiter korrigiert werden, wenn man die Mittelwertbildung bei hoher Verstärkung weiterlaufen läßt.

Gemäß Fig. 2 wird das Signal für die Sendespule 18 mit einem Kurvenformer 40 erzeugt. Wenn man aber aus Leistungsgründen mit einem Klasse D-Verstärker arbeiten möchte, ist es zweckmäßiger, über einen sequentiellen Digitalspeicher direkt das Pulsdauermodulations (PDM)-Signal auszugeben. Die Sendefrequenz wird dann ausschließlich über die Frequenz der Ausgaberate gesteuert. Wenn die Spule 18 mit einem geeigneten PDM-Signal angesteuert wird, emittiert sie ein sinusförmiges Feld. Das hat den Vorteil, dass keine Oberwellen emittiert werden und die Empfindlichkeit der Messvorrichtung stärker auf die Sendefrequenz konzentriert wird, also die Messvorrichtung in ihrer Qualität verbessert wird.

Statt wie in Fign. 3 und 4 gezeigt eine "Normaldifferenzspule" zu verwenden, die zwei subtraktiv geschaltete Spulen umfaßt, könnte man auch eine Multidifferenzsspule verwenden, die dann beispielsweise vier Empfängerspulen aufweist, wobei jede der beiden Empfängerspulen 12 und 14 der Normaldifferenzspule durch zwei gegeneinander geschaltete Empfängerspulen ersetzt ist. Eine Multidifferenzspule hat eine bessere Rauschunterdrückung, d.h. ein besseres Signal-Rausch-Verhältnis, als eine Normaldifferenzspule, und die Signalform ist ausgeprägter. Allerdings ist der Aufbau aufwändiger und die Signalamplituden sind kleiner. Auch erhält man gegebenenfalls störende Vor- und Nachschwinger.

Im folgenden soll das von der Differenzspule beim Durchgang eines Partikels erzeugte Signal als "Differenzsignal" bezeichnet werden.

Die Größe der erfassten Partikel liegt typischerweise zwischen 1 und 25 μm. Größere Parttikel werden üblicherweise aus dem Schmiermittel ausgefiltert, um Schäden an der Maschine zu verhindern.

Zweckmäßigerweise wird die Zahl der erfassten Partikel pro Zeit ermittelt, woraus die Konzentration der elektrisch leitenden Partikel in der Flüssigkeit ermittelt werden kann, da der Flüssigkeitsdurchsatz üblicherweise bekannt und im wesentlichen konstant ist.

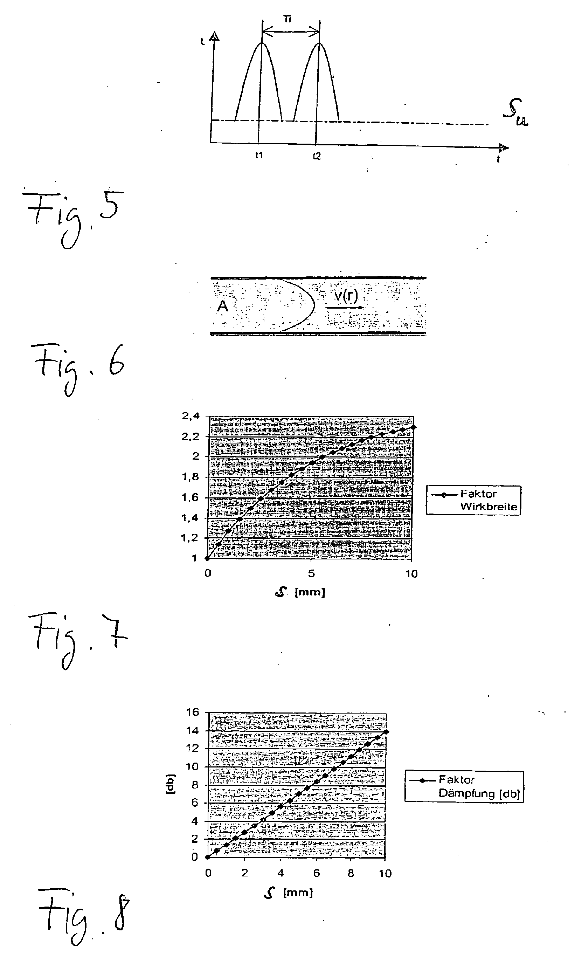

Aus der Auswertung des Differenzsignals der Empfängerspulen kann nicht nur der Durchgang eines Partikels erfasst werden, sondern es können auch zusätzliche Informationen gewonnen werden, insbesondere bezüglich der radialen Position des Partikels beim Durchgang durch die Empfängerspulen, d.h. dem radialen Abstand des Partikels von der Wand der Empfängerspulen, der Fließgeschwindigkeit des erfassten Partikels, der Größe des erfassten Partikels sowie des Volumenstroms, d.h. der über den Querschnitt des Rohrstücks 10 gemittelte Strömungsgeschwindigkeit der Flüssigkeit 16. Wie solche zusätzlichen Information gewonnen werden kann, soll im folgenden anhand der Fign. 5 bis 8 beispielhaft erläutert werden.

In der Regel besteht bei einem Partikelzähler auch der Wunsch, den Volumenstrom zu erfassen, um die gezählten Partikel auf ein Volumen normieren zu können (Partikel/*ml*) und bestehenden Normtabellen zuzuordnen. Dabei muss bei gegebenem Rohrdurchmesser die Geschwindigkeit der Flüssigkeit gemessen werden, woraus man dann das Volumen des Schmiermittels berechnen kann, das während der Messzeit (typischerweise 1 bis 30 Minuten) den Partikelzähler passiert hat. Während es hierzu grundsätzlich viele unterschiedliche - mehr oder weniger aufwändige - Lösungen, z.B. basierend auf Temperaturmessung, Ultraschall, Impulse aus mechanischen Mühlrädchen, etc., gibt, ist es besonders vorteilhaft, den Volumenstrom aus den für die Partikelzählung ohnehin erfassten Wirbelstromsignalen abzuleiten. Dadurch kann eine Lösung ohne zusätzliche Sensorik realisiert werden, was zu geringeren Kosten, geringerer Ausfallwahrscheinlichkeit und geringerem Platzbedarf führt. Zwar kann man in diesem Fall die Fließgeschwindigkeit nur dann erfassen, wenn Partikel erkannt werden. Dies ist jedoch in der Regel kein Problem, da ohnehin eine Messung nur stattfindet, wenn auch Partikel erkannt werden.

Ferner ist es bei einem Partikelzähler üblicherweise von Vorteil, die Größe der erfassten Partikel abzuschätzen und die erfassten Partikel nach der abgeschätzten Größe zu klassifizieren, um eine möglichst aussagekräftige Charakterisierung des Maschinenzustands zu erzielen. Beispielsweise kann bei über Überschreiten eines vorgegebenen Grenzwerts für die Zahl der insgesamt oder für eine bestimmte Größenklasse erfassten Partikel pro Zeit ein Alarmsignal ausgegeben werden.

Da sowohl die Amplitude als auch der zeitliche Verlauf des von einem Partikel verursachten Differenzsignals von der radialen Position des Partikels im Rohrstück 10 bzw. in der Spule abhängt, ist es sowohl für die Geschwindigkeitsmessung bzw. Volumenstrommessung als auch für die Partikelgrößenmessung vorteilhaft, die radiale Position des Partikels abzuschätzen und die Geschwindigkeitsmessung bzw. die Größenmessung entsprechend zu korrigieren.

In Fig. 5 ist ein Beispiel für den idealisierten Verlauf des Betrags des Differenzsignals einer Normaldifferenzspule, wie z.B. der von den Empfängerspulen 12, 14 von Fig. 3 gebildeten Differenzspule, gezeigt, wobei die Messwerte unterhalb eines Amplitudenschwellwerts $S_u$ abgeschnitten wurden (das abgeschnittene Signal umfasst Grundrauschen sowie Anfang und Ende des Differenzsignals). Solange die Signalamplitude oberhalb des Schwellwerts $S_u$ liegt, wird das Signal aufgezeichnet und im Prozessor der Auswenteeinheit gespeichert. Man erhält zwei getrennte Signalbögen, die jeweils ein Maximum beim Zeitpunkt $t_1$ bzw. $t_2$ aufweisen.

Das jeweilige Amplitudenmaximum, d.h. der jeweilige Zeitpunkt $t_1$ bzw. $t_2$, kann beispielsweise durch parabolische Ausgleichsrechnung oder einfacher durch eine Maximalwertsuche bestimmt werden. Entscheidend für die nachfolgende Auswertung ist der Wert des Amplitudenmaximums $A_i$ sowie die Zeitdifferenz $T_i$, die sich aus der Differenz zwischen $t_1$ und $t_2$ ergibt. Für jedes Zählereignis, d.h. für jeden gefundenen Partikel $i$, wird die entsprechende Maximalamplitude $A_i$ des Differenzsignals (ggf. auch komplex) und die dazugehörige Zeitdifferenz $T_i$ abgespeichert.

Da die beiden Differenzspulen 12, 14 in axialer Richtung einen Abstand $d_{sp}$ aufweisen, ist die Zeitdifferenz $T_i$ in erster Näherung proportional zur Strömungsgeschwindigkeit $v_i$ des Partikels. Der axiale Abstand der Spulen schlägt sich dabei in der sogenannten Wirkbreite der Differenzspule nieder. Grundsätzlich gilt der Zusammenhang $v_i = k*WB/T_i$. Der Faktor $k$ hängt von bestimmten Eigenschaften der Differenzspule ab und kann im Werk einmalig für den jeweiligen Typ des Partikelzählers ermittelt werden.

Die Wirkbreite $WB$ hängt von der radialen Position des Partikels beim Durchgang durch die Differenzspule ab, wobei sie mit zunehmendem radialem Abstand des Partikels von der Spulenwand zunimmt. Dieser Tatsache kann Rechnung getragen werden, indem man werksseitig für den jeweiligen Typ des Partikelzählers die Abhängigkeit der Wirkbreite von der radialen Position des Partikels empirisch ermittelt. In Fig. 7 ist ein Beispiel für die relative Vergrößerung der Wirkbreite mit zunehmendem radialen Partikelabstand $s$ gezeigt. Anhand einer solchen empirischen Kurve kann, wie dies nachstehend genauer beschrieben wird, die gemessene Zeitdifferenz $T_i$ hinsichtlich des radialen Abstands des Partikels $i$ korrigiert werden.

Grundsätzlich ist die Maximalamplitude $A_i$ des Differenzsignals ein Maß für die Größe des Partikels i. Jedoch ist hierbei zu berücksichtigen, dass die Dämpfung der Differenzspule von der radialen Position $s$ des Partikels abhängt, so dass für eine verlässliche Abschätzung der Partikelgröße die gemessene Maximalamplitude $A_i$ entsprechend korrigiert werden muss. Dies kann dadurch erfolgen, dass die Abhängigkeit der Dämpfung des Differenzsignals vom radialen Abstand $s$ von der Spulenwand für den jeweiligen Typ des Partikelzählers werksseitig empirisch ermittelt wird. In Fig. 8 ist ein Beispiel für eine solche Korrekturkurve gezeigt. Je weiter ein Partikel vom Spulensystem radial entfernt ist, um so schwächer wird die Signalamplitude. Zwar spielt auch hier die Größe des Partikels eine gewisse Rolle; die Dämpfungsfunktion bezüglich des Abstands wird aber im wesentlichen gleich verlaufen.

Ferner ist bezüglich der Teilchengeschwindigkeiten noch zu berücksichtigen, dass die Geschwindigkeit einer laminaren Strömung in einem Rohr in bekannter Weise vom radialen Abstand r von der Rohrwand abhängt, wobei die Abhängigkeit parabolisch ist und die maximale Strömungsgeschwindigkeit im Zentrum des Rohrs liegt. Man wird also aus diesem Grund eine bestimmte Verteilung der Partikelgeschwindigkeiten und somit der gemessenen Zeitdifferenzen $T_i$ erhalten.

Bei der Auswertung der Differenzsignale ist es zweckmäßig, folgende Annahmen zu machen:

1. Während eines Messintervalls von typischerweise 1 bis 30 Minuten bleibt der Volumenstrom im wesentlichen konstant, wobei dies natürlich auch von der jeweiligen Anlage abhängt. In der Praxis treten Geschwindigkeitsschwankungen im wesentlichen beim Anfahren der Anlage auf. In dieser Zeit wird aber ohnehin keine Partikelmessung durchgeführt. Ansonsten ergeben sich Änderungen des Volumenstroms hauptsächlich durch Schwankungen der Umgebungstemperatur (Einfluss auf die Viskosität des Schmiermittels) und durch die sich langsam verändernde Durchlässigkeit der Schmiermittelfilter. Nur bei Störfällen kann es zu schnellen Änderungen der Fließgeschwindigkeit, wenn z.B. ein Filter bricht.

2. Der radiale Abstand *s* der Partikel zum Spulensystem ist statistisch gesehen gleich verteilt.

3. Es tritt keine turbulente Strömung auf. Dies kann durch Leitelemente sichergestellt werden.

4. Alle Partikel sind in ihrer Ausdehnung viel kleiner als die Wirkbreiten der Spulen.

Im folgenden wird ein Beispiel für die Auswertung der Differenzsignale beschrieben.

Wie bereits erwähnt, werden während eines Messintervalls von typischerweise 1 bis 30 Minuten die Maximalamplitude $A_i$ und die Zeitdifferenz $T_i$ für jeden erfassten Partikel *i* gespeichert. Um eine verlässliche Auswertung zu ermöglichen, sollte eine gewisse Mindestanzahl von Partikeln erfasst werden. Gegebenenfalls muss bei kleiner Partikelkonzentration das Messintervall entsprechend verlängert werden. Als Ergebnis erhält man eine gewisse Verteilung der Maximalamplituden und der Zeitdifferenzen. Die kleinsten Zeitdifferenzen $T_i$ repräsentieren dabei Partikel im Zentrum der Spulenanordnung, d.h. $s = r_0$. Für die Verteilung der Strömungsgeschwindigkeiten $v(r)$ einer laminaren Strömung in einem Rohr gilt:

$$v(r) = \frac{\Delta p}{4 \cdot l \cdot \eta} \cdot (r_0{}^2 - r^2)$$

Dabei ist $\Delta p$ die Druckdifferenz in Pascal, *l* ist die Länge des Rohrs in Meter und $\eta$ ist die kinematische Viskosität in Pa s.

Die tatsächliche Maximalgeschwindigkeit im Rohr kann abgeschätzt werden, indem man die kleinste gemessene Zeitdifferenz $T_i$ mit dem "Faktor Wirkbreite" für $r_0$ dividiert:

$$T' = Min\ (T_i)/FW(r_0)$$

Da wir den tatsächlichen geometrischen Spulenabstand $d_{Sp}$ und jetzt auch die korrigierte Zeit $T'$ und kennen, kann die Maximalgeschwindigkeit $v_{max}$ berechnet werden:

$$v_{max} = d_{Sp}/T'$$

Daraus ergibt sich für die mittlere Geschwindigkeit $v_{mean} = v_{max}/2$. Für den Volumenstrom gilt:

$$I = v_{mean}\ r_0{}^2 \pi$$

Da man nun $v_{max}$ kennt, kann die Konstante $\Delta p/l\ \eta$ bestimmt werden:

$$c = \frac{\Delta p}{l \cdot \eta} = \frac{4 \cdot v_{max}}{r_o{}^2}$$

Somit ist nun die tatsächliche Geschwindigkeitsverteilung $v(r)$ bzw. $v(s)$ im Rohr bekannt. Damit kann man nun korrigierte Zeitdifferenzen $T'_i(s)$ entsprechend einer berechneten Wirkbreite bestimmen:

$$T'(s) = \frac{d_{Sp}}{v(s) \cdot FW(s)} = \frac{d_{Sp}}{\frac{c}{4}\left(r_o{}^2 - (r_o - s)^2\right) \cdot FW(s)}$$

Damit erhält man nun beispielsweise eine Tabelle mit den Werten $T'_i(s)$ in Abhängigkeit vom radialen Abstand $s$ des Partikels $i$ zur Spule. Im einfachsten Fall kann man diese Tabelle als Look-up-Table benutzen, um jedem gefundenen Partikel $i$ einen radialen Abstand $s$ zur Spule zuzuordnen. Dabei nimmt man den gemessenen Wert $T_i$ und sucht nach dem nächstgelegenen Wert $T'_i(s)$ in der Tabelle.

Mittels einer solchen Abstandszuordnung kann man nicht nur die gemessene Zeitdifferenz und damit die berechnete Partikelgeschwindigkeit korrigieren, sondern man kann auch die jeweils gemessene Maximalamplitude $A_i$ anhand der vorab empirisch ermittelten Abhängigkeit der Differenzsignaldämpfung vom radialen Partikelabstand $s$ korrigieren. Dabei wird der Amplitudenwert im einfachsten Fall auf einen Skalar reduziert, zweckmäßigerweise auf den Maximalwert der Betragsdarstellung des Differenzsignals von Fig. 5. Dieser Wert wird dann mit dem dazugehörigen Dämpfungswert korrigiert. Anschließend wird der Wert anhand der Bewertungsschwellen (z.B. acht Bewertungsschwellen) bewertet. Jeder Bewertungsbereich entspricht dabei einem Größenbereich der Partikel. Für jeden Bereich gibt es einen Zähler, der inkrementiert wird, wenn die gemessene Partikelamplitude in diesen Bereich fällt. Nach dem Messintervall wird das Gesamtvolumen der Flüssigkeit anhand der Messdauer und dem ermittelten Volumenstrom berechnet und entsprechend der Zählerstände eine Verunreinigungsklasse ausgewiesen, z.B. nach ISO 4406. Alternativ kann die Amplitudenauswertung anhand eines dem jeweiligen Partikel anhand des Differenzsignals zugeordneten Vektors erfolgen (dabei wird dann nicht nur der maximale Amplitudenwert sondern auch die Phase berücksichtigt).

Es versteht sich, dass die empirisch ermittelten Korrekturfunktionen gemäß Fig. 7 und Fig. 8 mittels Ausgleichrechnung durch geeignete Funktionen, z.B. genäherte Parabeln sowie deren Umkehrfunktion, dargestellt werden können. In diesem Fall kann der Look-up-Table entfallen.

Unmittelbar nach dem Einschalten des Partikelzählers ist die Strömungsgeschwindigkeit noch nicht bekannt ist und somit können Partikel dann u.U. nicht zuverlässig diskretisiert werden, da die Zuordnung der einzelnen Betragssignalbögen zum Differenzsignal ohne Kenntnis des zu erwartenden Bereichs der Zeitdifferenzen $T_i$ zu einem bestimmten Ereignis, nämlich dem Durchgang eines Partikels, nicht immer zuverlässig möglich ist. Um diese Probleme zu umgehen, kann als "Anfahrhilfe" ein Signal nach dem Überschreiten des unteren Schwellwerts $S_u$ in einer Länge aufgezeichnet werden, die für die Erkennung einer Mindestströmungsgeschwindigkeit ausreichend ist. Die erfaßten Differenzsignale können dann anhand eines solchermaßen erfaßten typischen Verlaufs separiert bzw. einzelnen Partikeldurchgängen zugeordnet werden. Eine solche Separation kann beispielsweise mittels Kreuzkorrelation mit Variation der vorgegebenen Wirkbreiten bzw. Zeitdifferenzen $T_i$ erfolgen. Dabei wird die vorgegebene Wirkbreite bzw. die vorgegebene Zeitdifferenz so variiert, dass die Amplituden der Kreuzkorrelationsfunktion maximiert werden.

## Patentansprüche

1. Verfahren zum Erfassen von elektrisch leitenden Partikeln (20) in einer in einem Rohrstück (10) mit einer Geschwindigkeit (v) strömenden Flüssigkeit (16), wobei die Flüssigkeit mittels einer Senderspule (18) mit periodischen elektromagnetischen Wechselfeldern beaufschlagt wird, um Wirbelströme in den Partikeln zu induzieren, wobei mittels einer durch eine Wirkbreite (WB) charakterisierte und als Spulenanordnung (12, 14, 15) ausgebildete Sonde ein periodisches elektrisches Signal entsprechend der Wirbelströme erfasst wird, das eine Trägerschwingung aufweist, deren Amplitude und/oder Phase durch die Partikel moduliert wird, wenn die Partikel in die Wirkbreite der Spulenanordnung gelangen, wobei

   das Sondensignal mittels einer frequenzselektiven ersten Filtereinheit (19) gefiltert wird,

   das mittels der ersten Filtereinheit gefilterte Signal mittels einer triggerbaren A/D-Wandlerstufe (35) abgetastet wird, um ein demoduliertes digitales Messsignal zu erhalten,

   das digitale Messsignal mittels einer digitalen frequenzselektiven, einstellbaren zweiten Filtereinheit (52) gefiltert wird, um ein Nutzsignal zu gewinnen, und

   das Nutzsignal ausgewertet wird, um den Durchgang von elektrisch leitenden Partikeln in dem Rohrstück zu erfassen, wobei die A/D-Wandlerstufe mit einem $n$-ten, ganzzahligen Bruchteil der Frequenz der Trägerschwingung getriggert wird, wobei n in Abhängigkeit von der Partikelfrequenz, die sich als Quotient aus der Strömungsgeschwindigkeit der Flüssigkeit und der Wirkbreite der Spulenanordnung ergibt, gewählt wird, und wobei die frequenzselektive zweite Filtereinheit in Abhängigkeit von der Partikelfrequenz eingestellt wird.

2. Verfahren gemäß Anspruch 1, **dadurch gekennzeichnet, dass** das Rohrstück (10) Teil eines Schmiermittel-Kreislaufs ist.

3. Verfahren gemäß Anspruch 2, **dadurch gekennzeichnet, dass** es sich bei der Flüssigkeit (16) um Schmieröl einer Maschine handelt.

4. Verfahren gemäß einem der vorhergehenden Ansprüche, **dadurch gekennzeichnet, dass** die Sendefrequenz der Senderspule (18) zwischen 20 kHz und 500 kHz liegt.

5. Verfahren gemäß einem der vorhergehenden Ansprüche, **dadurch gekennzeichnet, dass** die Größe der erfassten Partikel (20) zwischen 1 und 25 $\mu$m liegt.

6. Verfahren gemäß einem der vorhergehenden Ansprüche, **dadurch gekennzeichnet, dass** die Zahl der erfassten Partikel (20) pro Zeit ermittelt wird.

7. Verfahren gemäß einem der vorhergehenden Ansprüche, **dadurch gekennzeichnet, dass** die Konzentration der elektrisch leitenden Partikel (20) in der Flüssigkeit (16) ermittelt wird.

8. Verfahren gemäß einem der vorhergehenden Ansprüche, **dadurch gekennzeichnet, dass** die Senderspule (18) mit einer Wechselspannung versorgt wird, um die periodischen elektromagnetischen Wechselfelder zu erzeugen, wobei die Wechselspannung aus einem binären Signal durch Kurvenformung erzeugt wird, oder dass die Senderspule durch Ansteuerung mit einem PDM-Signal veranlasst wird, ein sinusförmiges Feld zu emittieren.

9. Verfahren gemäß Anspruch 8, **dadurch gekennzeichnet, dass** das Triggersignal für die A/D-Wandlerstufe (35) dadurch erzeugt wird; dass die Frequenz des für die Erzeugung der Wechselspannung für die Senderspule (18) verwendeten binären Signals durch n geteilt wird.

10. Verfahren gemäß einem der vorhergehenden Ansprüche, **dadurch gekennzeichnet, dass** n umgekehrt proportional zu der Partikel frequenz gewählt wird, um die Triggerrate der A/D-Wandlerstufe (35) mindestens annähernd proportional zu der Partikelfrequenz zu wählen.

11. Verfahren gemäß einem der vorhergehenden Ansprüche, **dadurch gekennzeichnet, dass** n so gewählt wird, dass in ein Zeitintervall, das dem Inversen der Partikelfrequenz entspricht, mindestens 5, vorzugsweise mindestens 20, Abtastungen durch die A/D-Wandlerstufe (35) fallen.

12. Verfahren gemäß einem der vorhergehenden Ansprüche, **dadurch gekennzeichnet, dass** n so gewählt wird, dass in ein Zeitintervall, das dem Inversen der Partikelfrequenz entspricht, höchstens 100, vorzugsweise höchstens 50, Abtastungen durch die A/D-Wandlerstufe (35) fallen.

13. Verfahren gemäß einem der vorhergehenden Ansprüche, **dadurch gekennzeichnet, dass** das Einstellen der frequenzselektiven zweiten Filtereinheit (52) in Abhängigkeit von der Partikelfrequenz selbsttätig dadurch erfolgt, dass die zweite Filtereinheit mit der Abtastrate der A/D-Wandlerstufe (35) getaktet wird.

14. Verfahren gemäß einem der vorhergehenden Ansprüche, **dadurch gekennzeichnet, dass** die zweite Filtereinheit (52) einen Tiefpass aufweist, um Störanteile des demodulierten digitalen Signals mit Frequenzen oberhalb der Partikelfrequenz auszublenden, wobei die Eckfrequenz des Tiefpasses über der Partikelfrequenz, vorzugsweise über dem Zweifachen der Partikelfrequenz, liegt.

15. Verfahren gemäß einem der vorhergehenden Ansprüche, **dadurch gekennzeichnet, dass** die zweite Filtereinheit (52) einen Hochpass aufweist, um Gleichanteile des demodulierten digitalen Signals auszublenden, wobei die Eckfrequenz des Hochpasses unter der Partikelfrequenz, vorzugsweise unter einem Viertel der Partikelfrequenz, liegt.

16. Verfahren gemäß einem der vorhergehenden Ansprüche, **dadurch gekennzeichnet, dass** die Frequenz der Trägerschwingung so gewählt wird, dass sie mindestens das Zehnfache der Partikelfrequenz beträgt.

17. Verfahren gemäß einem der vorhergehenden Ansprüche, **dadurch gekennzeichnet, dass** die A/D-Wandlerstufe (35), wenn sie getriggert wird, um eine feste Phasendifferenz versetzt zwei Werte abtastet, um das digitale Messsignal als zweikomponentiges Signal zu erhalten.

**18.** Verfahren gemäß Anspruch 17, **dadurch gekennzeichnet, dass** die Phasendifferenz 90° oder $m * 360° + 90°$ ist, wobei $m$ eine ganze Zahl ist.

**19.** Verfahren gemäß Anspruch 17 oder 18, **dadurch gekennzeichnet, dass** die beiden Komponenten des von der A/D-Wandlerstufe (35) gelieferten digitalen Messsignal separat mittels der zweiten Filtereinheit (52) gefiltert werden, um das Nutzsignal als zweikomponentiges Signal zu gewinnen.

**20.** Verfahren gemäß Anspruch 19, **dadurch gekennzeichnet, dass** bei der Auswertung des Nutzsignals beide Komponenten berücksichtigt werden.

**21.** Verfahren gemäß einem der vorhergehenden Ansprüche, **dadurch gekennzeichnet, dass** die Beaufschlagung der Flüssigkeit (16) mittels der Senderspule (18) mit den elektromagnetischen Wechselfeldern mindestens für einen Teil eines jeden Intervalls zwischen zwei aufeinander folgenden Triggersignalen für die A/D-Wandlerstufe (35) unterbrochen wird.

**22.** Verfahren gemäß einem der vorhergehenden Ansprüche, **dadurch gekennzeichnet, dass** die erste Filtereinheit (19) mindestens einen als Aliasingfilter bzgl. der Abtastung durch die A/D-Wandlerstufe (35) wirkenden Tiefpass aufweist.

**23.** Verfahren gemäß einem der vorhergehenden Ansprüche, **dadurch gekennzeichnet, dass** die erste Filtereinheit (19) einen Hochpass aufweist, um niederfrequente Störsignale auszublenden.

**24.** Verfahren gemäß einem der vorhergehenden Ansprüche, **dadurch gekennzeichnet, dass** die Strömungsgeschwindigkeit (v) der Flüssigkeit (16) durch Messung bestimmt oder als Parameter fest vorgegeben wird.

**25.** Verfahren gemäß einem der vorhergehenden Ansprüche, **dadurch gekennzeichnet, dass** der A/D-Wandlerstufe (35) ein steuerbarer Verstärkers (17) vorgeschaltet ist, um das Signal auf die für die A/D-Wandlerstufe optimal geeignete Amplitude zu bringen.

**26.** Verfahren gemäß einem der vorhergehenden Ansprüche, **dadurch gekennzeichnet, dass** die Senderspule (18) das Rohrstück (10) umgibt, wobei die Spulenanordnung (15) mindestens eine erste das Rohrstück umgebende induktive Empfängerspule (12) und eine zweite das Rohrstück umgebende, axial zur ersten Empfängerspule beabstandete, induktive Empfängerspule (14) aufweist, die im Bereich der Senderspule angeordnet sind und subtraktiv geschaltet sind, um ein Differenzsignal entsprechend den von der Senderspule induzierten Wirbelströmen auszugeben, wobei die Senderspule die Primärseite und die Empfängerspulen die Sekundärseite einer transformatorischen Anordnung bilden, und wobei das Nutzsignal von dem Differenzsignal gebildet wird.

**27.** Verfahren gemäß Anspruch 26, **dadurch gekennzeichnet, dass** nur Differenzsignale bei der Partikelerfassung verwendet werden, deren Amplitude einen bestimmten Schwellwert überschreitet.

**28.** Verfahren gemäß Anspruch 27, **dadurch gekennzeichnet, dass** das Differenzsignal aufgezeichnet wird, solange die Signalamplitude den Schwellwert überschreitet.

**29.** Verfahren gemäß einem der Ansprüche 26 bis 28, **dadurch gekennzeichnet, dass** der radiale Abstand der erfassten Partikel (20) von der Empfängerspulenwand aus dem Differenzsignal abgeschätzt wird.

**30.** Verfahren gemäß einem der Ansprüche 26 bis 29, **dadurch gekennzeichnet, dass** dass die Strömungsgeschwindigkeit der erfassten Partikel (20) aus dem Differenzsignal abgeschätzt wird.

**31.** Verfahren gemäß Anspruch 30, **dadurch gekennzeichnet, dass** die Strömungsgeschwindigkeit der erfassten Partikel (20) aus dem zeitlichen Abstand der Extremwerte des Differenzsignals abgeschätzt wird.

**32.** Verfahren gemäß Anspruch 31, **dadurch gekennzeichnet, dass** die Strömungsgeschwindigkeit der erfassten Partikel (20) aus dem zeitlichen Abstand der Maxima des Betrags des Differenzsignals abgeschätzt wird.

**33.** Verfahren gemäß Anspruch 31 oder 32, **dadurch gekennzeichnet, dass** die Verteilung der ermittelten zeitlichen Abstände bestimmt wird und einer statistischen Analyse unterzogen wird.

**34.** Verfahren gemäß Anspruch 33, **dadurch gekennzeichnet, dass** bei der statistischen Analyse der ermittelten zeitlichen Abstände die theoretische radiale Verteilung der Strömungsgeschwindigkeit in einer laminaren Strömung berücksichtigt wird.

**35.** Verfahren gemäß Anspruch 34, **dadurch gekennzeichnet, dass** aus dem Maximum der abgeschätzten Partikelgeschwindigkeiten unter Berücksichtigung des geometrischen Abstands der Empfängerspulen die Strömungsgeschwindigkeit der Flüssigkeit (16) ermittelt wird.

**36.** Verfahren gemäß Anspruch 29, **dadurch gekennzeichnet, dass** der abgeschätzte radiale Abstand der erfassten Partikel (20) von der Empfängerspulenwand bei der Abschätzung der Strömungsgeschwindigkeit der Partikel verwendet wird.

**37.** Verfahren gemäß Anspruch 36, **dadurch gekennzeichnet, dass** vorab die Abhängigkeit der Wirkbreite der Empfängerspulen (12, 14) vom radialen Abstand der erfasstem Partikels (20) von der Empfängerspulenwand empirisch ermittelt wird.

**38.** Verfahren gemäß Anspruch 37, **dadurch gekennzeichnet, dass** bei der Abschätzung des radialen Abstands der erfassten Partikel (20) von der Empfängerspulenwand die theoretische radiale Verteilung der Strömungsgeschwindigkeit in einer laminaren Strömung, der geometrische Abstand der Empfängerspulen (12, 14) sowie die vorab ermittelte Abhängigkeit der Wirkbreite der Empfängerspulen vom radialen Abstand der Partikel berücksichtigt werden.

**39.** Verfahren gemäß einem der Ansprüche 26 bis 38, **dadurch gekennzeichnet, dass** die Größe der erfassten Partikel (20) aus dem Differenzsignal abgeschätzt wird und die erfassten Partikel nach der abgeschätzten Größe klassifiziert werden.

**40.** Verfahren gemäß Anspruch 39, sofern auf Anspruch 29 rückbezogen, **dadurch gekennzeichnet, dass** der abgeschätzte radiale Abstand jedes erfassten Partikel (20) von der Empfängerspulenwand bei der Abschätzung der Größe der Partikel verwendet wird.

**41.** Verfahren gemäß Anspruch 40, **dadurch gekennzeichnet, dass** vorab die Abhängigkeit der Amplitude des Differenzsignals vom radialen Abstand des Partikels (20) von der Empfängerspulenwand empirisch ermittelt wird.

**42.** Verfahren gemäß Anspruch 41, **dadurch gekennzeichnet, dass** bei der Abschätzung der Größe der erfassten Partikel (20) die theoretische radiale Verteilung der Strömungsgeschwindigkeit in einer laminaren Strömung, der geometrische Abstand der Empfängerspulen (12, 14), die vorab ermittelte Abhängigkeit der Wirkbreite der Empfängerspulen vom radialen Abstand der Partikel von der Empfängerspulenwand sowie die vorab ermittelte Abhängigkeit der Amplitude des Differenzsignals vom radialen Abstand der Partikel von der Empfängerspulenwand berücksichtigt werden.

**43.** Verfahren gemäß einem der vorhergehenden Ansprüche, **dadurch gekennzeichnet, dass** das Sondensignal, bevor es der triggerbaren A/D-Wandlerstufe (35) zugeführt wird, mit einer Offset-Spannung zur Kompensation einer Spulenfehlspannung beaufschlagt wird.

**44.** Verfahren gemäß Anspruch 43, **dadurch gekennzeichnet, dass** die Offset-Spannung mittels eines D/A-Wandlers erzeugt wird, wobei sich der Wert der Offset-Spannung aus einer Mittelwertbildung des demodulierten digitalen Messsignals ergibt.

**45.** Verfahren gemäß Anspruch 44, **dadurch gekennzeichnet, dass** die Mittelwertbildung stattfindet, während das Sondensignal mit der Offset-Spannung beaufschlagt wird.

**46.** Verfahren gemäß Anspruch 44, **dadurch gekennzeichnet, dass** die Mittelwertbildung stattfindet, bevor das Sondensignal mit der Offset-Spannung beaufschlagt wird.

**Claims**

**1.** Method for detecting electrically conductive particles (20) in a liquid (16), which flows in a piece of pipe (10) at a speed (v), wherein periodic alternating electromagnetic fields are applied to the liquid using a transmitter coil (18) in order to induce eddy currents in the particles, wherein a probe, which is **characterized by** an effective width (WB) and is in the form of a coil arrangement (12, 14, 15), is used to detect a periodic electric signal according to the eddy currents which has a carrier oscillation, the amplitude and/or phase of which is modulated by the particles if the particles pass within the effective width of the coil arrangement, wherein

the probe signal is filtered using a frequency-selective first filter unit (19),

the signal which is filtered using the first filter unit is sampled using a triggerable A/D converter stage (35) in order to obtain a demodulated digital measurement signal,

the digital measurement signal is filtered using a digital frequency-selective, adjustable second filter unit (52) in order to obtain a useful signal, and

the useful signal is evaluated in order to detect the passing of electrically conductive particles in the piece of pipe, wherein the A/D converter stage is triggered by an nth integer fraction of the frequency of the carrier oscillation, n being chosen as a function of the particle frequency, which is obtained as the quotient of the flow speed of the liquid and the effective width of the coil arrangement, and wherein the frequency-selective second filter unit is adjusted as a function of the particle frequency.

2. Method according to Claim 1, **characterized in that** the piece of pipe (10) is part of a lubrication circuit.

3. Method according to Claim 2, **characterized in that** the liquid (16) is a lubricant oil in a machine.

4. Method according to one of the preceding claims, **characterized in that** the transmission frequency of the transmitter coil (18) is between 20 kHz and 500 kHz.

5. Method according to one of the preceding claims, **characterized in that** the size of the detected particles (20) is between 1 and 25 $\mu$m.

6. Method according to one of the preceding claims, **characterized in that** the number of the detected particles (20) is ascertained per unit time.

7. Method according to one of the preceding claims, **characterized in that** the concentration of the electrically conductive particles (20) in the liquid (16) is ascertained.

8. Method according to one of the preceding claims, **characterized in that** the transmitter coil (18) is supplied with an alternating voltage in order to generate the periodic alternating electromagnetic fields, with the alternating voltage being produced from a binary signal by way of curve shaping, or **in that** driving of the transmitter coil using a PDM signal causes the transmitter coil to emit a sinusoidal field.

9. Method according to Claim 8, **characterized in that** the trigger signal for the A/D converter stage (35) is produced by the frequency of the binary signal used to create the alternating voltage for the transmitter coil (18) being divided by n.

10. Method according to one of the preceding claims, **characterized in that** n is chosen to be inversely proportional to the particle frequency in order to choose the trigger rate of the A/D converter stage (35) as at least approximately proportional to the particle frequency.

11. Method according to one of the preceding claims, **characterized in that** n is chosen such that at least 5, preferably at least 20, sampling operations by the A/D converter stage (35) fall within a time interval which corresponds to the inverse of the particle frequency.

12. Method according to one of the preceding claims, **characterized in that** n is chosen such that at most 100, preferably at most 50, sampling operations by the A/D converter stage (35) fall within a time interval which corresponds to the inverse of the particle frequency.

13. Method according to one of the preceding claims, **characterized in that** the adjustment of the frequency-selective second filter unit (52) takes place automatically as a function of the particle frequency by the second filter unit being clocked at the sampling rate of the A/D converter stage (35).

14. Method according to one of the preceding claims, **characterized in that** the second filter unit (52) has a low-pass filter in order to remove interfering components of the demodulated digital signals with frequencies above the particle frequency, wherein the cut-off frequency of the low-pass filter is above the particle frequency, preferably above twice the particle frequency.

15. Method according to one of the preceding claims, **characterized in that** the second filter unit (52) has a high-pass filter in order to remove DC components of the demodulated digital signal, wherein the cut-off frequency of the high-pass filter is below the particle frequency, preferably below a quarter of the particle frequency.

16. Method according to one of the preceding claims, **characterized in that** the frequency of the carrier oscillation is chosen such that it is at least ten times the particle frequency.

17. Method according to one of the preceding claims, **characterized in that** the A/D converter stage (35), once triggered, samples two values in a manner offset by a fixed phase difference, in order to obtain the digital measurement signal as a two-component signal.

18. Method according to Claim 17, **characterized in that** the phase difference is 90° or m * 360° + 90°, with m being an integer.

19. Method according to Claim 17 or 18, **characterized in that** the two components of the digital measurement signal supplied by the A/D converter stage (35) are filtered separately using the second filter unit (52) in order to obtain the useful signal as a two-component signal.

20. Method according to Claim 19, **characterized in that** the two components are taken into account in the evaluation of the useful signal.

21. Method according to one of the preceding claims, **characterized in that** applying the alternating electromagnetic fields to the liquid (16) using the transmitter coil (18) is interrupted at least for a portion of each interval between two successive trigger signals for the A/D converter stage (35).

22. Method according to one of the preceding claims, **characterized in that** the first filter unit (19) has at least one low-pass filter which acts as an aliasing filter with respect to the sampling operation by the A/D converter stage (35).

23. Method according to one of the preceding claims, **characterized in that** the first filter unit (19) has a high-pass filter in order to remove low-frequency interfering signals.

24. Method according to one of the preceding claims, **characterized in that** the flow speed (v) of the liquid (16) is determined by way of measurement or is prespecified fixedly as a parameter.

25. Method according to one of the preceding claims, **characterized in that** a controllable amplifier (17) is connected upstream of the A/D converter stage (35) in order to bring the signal to the amplitude which is optimally suited to the A/D converter stage.

26. Method according to one of the preceding claims, **characterized in that** the transmitter coil (18) surrounds the piece of pipe (10), wherein the coil arrangement (15) has at least one first inductive receiver coil (12) surrounding the piece of pipe and a second inductive receiver coil (14) which surrounds the piece of pipe at an axial spacing from the first receiver coil, which receiver coils are arranged in the region of the transmitter coil and are connected subtractively in order to emit a difference signal corresponding to the eddy currents induced by the transmitter coil, with the transmitter coil forming the primary side and the receiver coils forming the secondary side of a transformer-based arrangement and with the useful signal being formed by the difference signal.

27. Method according to Claim 26, **characterized in that** only difference signals having an amplitude which exceeds a specific threshold value are used in the particle detection.

28. Method according to Claim 27, **characterized in that** the difference signal is recorded for as long as the signal amplitude exceeds the threshold value.

29. Method according to one of Claims 26 to 28, **characterized in that** the radial spacing of the detected particles (20) from the receiver coil wall is estimated from the difference signal.

30. Method according to one of Claims 26 to 29, **characterized in that** the flow speed of the detected particles (20) is estimated from the difference signal.

31. Method according to Claim 30, **characterized in that** the flow speed of the detected particles (20) is estimated from the time interval between the extreme values of the difference signal.

32. Method according to Claim 31, **characterized in that** the flow speed of the detected particles (20) is estimated from the time interval between maxima of the magnitude of the difference signal.

33. Method according to Claim 31 or 32, **characterized in that** the distribution of the ascertained time intervals is determined and subjected to a statistical analysis.

34. Method according to Claim 33, **characterized in that** the theoretical radial distribution of the flow speed in a laminar flow is taken into account in the statistical analysis of the ascertained time intervals.

35. Method according to Claim 34, **characterized in that** the flow speed of the liquid (16) is ascertained from the maximum of the estimated particle speeds, taking into account the geometric spacing of the receiver coils.

36. Method according to Claim 29, **characterized in that** the estimated radial spacing of the detected particles (20) from the receiver coil wall is used in the estimation of the flow speed of the particles.

37. Method according to Claim 36, **characterized in that** first the dependence of the effective width of the receiver coils (12, 14) on the radial spacing of the detected particles (20) from the receiver coil wall is empirically ascertained.

38. Method according to Claim 37, **characterized in that** the theoretical radial distribution of the flow speed in a laminar flow, the geometric spacing of the receiver coils (12, 14) and the dependence of the effective width of the receiver coils ascertained first on the radial spacing of the particles are taken into account in the estimation of the radial spacing of the detected particles (20) from the receiver coil wall.

39. Method according to one of Claims 26 to 38, **characterized in that** the size of the detected particles (20) is estimated from the difference signal and the detected particles are classified according to the estimated size.

40. Method according to Claim 39, where it refers back to Claim 29, **characterized in that** the estimated radial spacing of each detected particle (20) from the receiver coil wall is used in the estimation of the size of the particles.

41. Method according to Claim 40, **characterized in that** first the dependence of the amplitude of the difference signal on the radial spacing of the particle (20) from the receiver coil wall is empirically ascertained.

42. Method according to Claim 41, **characterized in that** the theoretical radial distribution of the flow speed in a laminar flow, the geometric spacing of the receiver coils (12, 14), the dependence of the effective width of the receiver coils ascertained first on the radial spacing of the particles from the receiver coil wall and the dependence of the amplitude of the difference signal ascertained first on the radial spacing of the particles from the receiver coil wall are taken into account in the estimation of the size of the detected particles (20).

43. Method according to one of the preceding claims, **characterized in that** the probe signal has applied to it, before it is supplied to the triggerable A/D converter stage (35), an offset voltage to compensate for an incorrect coil voltage.

44. Method according to Claim 43, **characterized in that** the offset voltage is produced using a D/A converter, with the value of the offset voltage being obtained from an averaging of the demodulated digital measurement signal.

45. Method according to Claim 44, **characterized in that** the averaging is carried out while the probe signal has the offset voltage applied to it.

46. Method according to Claim 44, **characterized in that** the averaging is carried out before the probe signal has the offset voltage applied to it.

**Revendications**

1. Procédé de détection de particules électriquement conductrices (20) dans un liquide (16) qui s'écoule dans une pièce tubulaire (10) à une vitesse (v),

   des champs électromagnétiques alternatifs étant appliqués périodiquement sur le liquide au moyen d'une bobine émettrice (18) pour induire des courants de Foucault dans les particules,

   un signal électrique périodique qui correspond aux courants de Foucault étant détecté au moyen d'une sonde **caractérisée par** une portée d'action (WB) et configurée comme ensemble de bobines (12, 14, 15),

   le signal présentant une oscillation porteuse dont l'amplitude et/ou la phase sont modulées par les particules lorsque les particules passent dans la portée d'action de l'ensemble de bobines,

   le signal de sonde étant filtré au moyen d'une première unité de filtrage (19) sélective en fréquence,

   le signal filtré au moyen de la première unité de filtrage étant échantillonné au moyen d'un étage de convertisseur A/N (35) asservi pour obtenir un signal numérique de mesure démodulé,

   le signal numérique de mesure étant filtré au moyen d'une deuxième unité de filtrage numérique (52), réglable et sélective en fréquence, pour obtenir un signal utile,

   le signal utile étant évalué pour détecter le passage de particules électriquement conductrices dans la pièce tubulaire,

   l'étage de conversion A/N étant activé par une fraction entière d'ordre n de la fréquence de l'oscillation porteuse, n étant sélectionné en fonction de la fréquence des particules qui est obtenue en tant que quotient de la vitesse d'écoulement du liquide et de la portée d'action de l'ensemble de bobines, la deuxième unité de filtrage sélective en fréquence étant réglée en fonction de la fréquence des particules.

2. Procédé selon la revendication 1, **caractérisé en ce que** la pièce tubulaire (10) fait partie d'un circuit de lubrifiant.

3. Procédé selon la revendication 2, **caractérisé en ce que** le liquide (16) est l'huile de lubrification d'une machine.

4. Procédé selon l'une des revendications précédentes, **caractérisé en ce que** la fréquence d'émission de la bobine d'émission (18) est située entre 20 kHz et 500 kHz.

5. Procédé selon l'une des revendications précédentes, **caractérisé en ce que** la taille des particules (20) détectées est comprise entre 1 et 25 $\mu$m.

6. Procédé selon l'une des revendications précédentes, **caractérisé en ce que** le nombre de particules (20) détectées par l'unité de temps est déterminé.

7. Procédé selon l'une des revendications précédentes, **caractérisé en ce que** la concentration des particules électriquement conductrices (20) dans le liquide (16) est déterminée.

8. Procédé selon l'une des revendications précédentes, **caractérisé en ce que** la bobine émettrice (18) est alimentée en une tension alternative pour produire les champs électromagnétiques alternatifs périodiques, la tension alternative étant formée à partir d'un signal binaire par formation d'une courbe, ou **en ce que** la bobine émettrice est amenée à émettre un champ sinusoïdal en étant commandée par un signal PDM.

9. Procédé selon la revendication 8, **caractérisé en ce que** le signal d'activation de l'étage (35) de conversion A/N est formé en divisant par n la fréquence du signal binaire utilisé pour produire la tension alternative pour la bobine émettrice (18).

10. Procédé selon l'une des revendications précédentes, **caractérisé en ce que** n est inversement proportionnel à la fréquence des particules de telle sorte que le taux d'activation de l'étage (35) de conversion A/N soit au moins approximativement proportionnel à la fréquence des particules.

11. Procédé selon l'une des revendications précédentes, **caractérisé en ce que** n est sélectionné de telle sorte qu'au moins 5 et de préférence au moins 20 échantillonnages par l'étage (35) de conversion A/N tombent dans un intervalle de temps qui correspond à l'inverse de la fréquence des particules.

12. Procédé selon l'une des revendications précédentes, **caractérisé en ce que** n est sélectionné de telle sorte qu'au plus 100 et de préférence au plus 50 échantillonnages par l'étage (35) de conversion A/N tombent dans un intervalle de temps qui correspond à l'inverse de la fréquence des particules.

**13.** Procédé selon l'une des revendications précédentes, **caractérisé en ce que** le réglage de la deuxième unité de filtrage (52) sélective en fréquence s'effectue automatiquement en fonction de la fréquence des particules en cadençant la deuxième unité de filtrage par le taux d'échantillonnage de l'étage (35) de conversion A/N.

**14.** Procédé selon l'une des revendications précédentes, **caractérisé en ce que** la deuxième unité de filtrage (52) présente un filtrage passe-bas qui bloque les parties perturbatrices du signal numérique démodulé à des fréquences supérieures à la fréquence des particules, la fréquence de coupure du filtrage passe-bas étant située au-dessus de la fréquence des particules et de préférence au-dessus du double de la fréquence des particules.

**15.** Procédé selon l'une des revendications précédentes, **caractérisé en ce que** la deuxième unité de filtrage (52) présente un filtrage passe-haut qui bloque les parties continues du signal numérique démodulé, la fréquence de coupure du filtrage passe-haut étant située en dessous de la fréquence des particules et de préférence en dessous du quart de la fréquence des particules.

**16.** Procédé selon l'une des revendications précédentes, **caractérisé en ce que** la fréquence de l'oscillation porteuse est sélectionnée de telle sorte qu'elle représente au moins dix fois la fréquence de particules.

**17.** Procédé selon l'une des revendications précédentes, **caractérisé en ce que** lorsque l'étage (35) de conversion A/N est activé, il échantillonne deux valeurs décalées par rapport à un déphasage fixe pour obtenir le signal numérique de mesure sous la forme d'un signal à deux composantes.

**18.** Procédé selon la revendication 17, **caractérisé en ce que** le déphasage est de 90° ou de m 360° + 90°, m étant un nombre entier.

**19.** Procédé selon les revendications 17 ou 18, **caractérisé en ce que** les deux composantes du signal numérique de mesure délivrées par l'étage (35) de conversion A/N sont filtrées séparément au moyen de la deuxième unité de filtrage (52) pour obtenir le signal utile sous la forme d'un signal à deux composantes.

**20.** Procédé selon la revendication 19, **caractérisé en ce que** les deux composantes sont prises en compte dans l'évaluation du signal utile.

**21.** Procédé selon l'une des revendications précédentes, **caractérisé en ce que** l'application de champs électromagnétiques alternatifs sur le liquide (16) au moyen de la bobine émettrice (18) est interrompue au moins pendant une partie de chaque intervalle qui s'écoule entre deux signaux successifs d'activation de l'étage (35) de conversion A/N.

**22.** Procédé selon l'une des revendications précédentes, **caractérisé en ce que** la première unité de filtrage (19) présente au moins un filtrage passe-bas qui agit comme filtre à bandes superposées pour l'échantillonnage par l'étage (35) de conversion A/N.

**23.** Procédé selon l'une des revendications précédentes, **caractérisé en ce que** la première unité de filtrage (19) présente un filtrage passe-haut qui bloque les signaux perturbateurs à basse fréquence.

**24.** Procédé selon l'une des revendications précédentes, **caractérisé en ce que** la vitesse d'écoulement (v) du liquide (16) est déterminée par mesure ou prédéterminée fixement comme paramètre.

**25.** Procédé selon l'une des revendications précédentes, **caractérisé en ce que** l'étage (35) de conversion A/N est précédée par un amplificateur asservi (17) qui amène le signal à l'amplitude qui convient le mieux pour l'étage de conversion A/N.

**26.** Procédé selon l'une des revendications précédentes, **caractérisé en ce que** la bobine émettrice (18) entoure la pièce tubulaire (10), l'ensemble de bobine (15) présentant au moins une première bobine inductive réceptrice (12) qui entoure la pièce tubulaire et une deuxième bobine inductive réceptrice (14) qui entoure la pièce tubulaire et qui est située à distance axiale de la première bobine réceptrice, les bobines réceptrices étant disposées dans la zone occupée par la bobine émettrice et étant raccordées soustractivement pour délivrer un signal de différence qui correspond aux courants de Foucault induits par la bobine émettrice, la bobine émettrice formant le côté primaire et les bobines réceptrices le côté secondaire d'un ensemble transformateur, le signal utile étant formé par le signal de différence.

EP 2 028 474 B1

27. Procédé selon la revendication 26, **caractérisé en ce que** pour la détection des particules, seuls sont utilisés des signaux de différence dont l'amplitude dépasse une valeur de seuil définie.

28. Procédé selon la revendication 27, **caractérisé en ce que** le signal de différence est enregistré tant que l'amplitude du signal dépasse une valeur de seuil.

29. Procédé selon l'une des revendications 26 à 28, **caractérisé en ce que** l'écartement radial entre les particules (20) détectées et la paroi de la bobine réceptrice est estimé à partir du signal de différence.

30. Procédé selon l'une des revendications 26 à 29, **caractérisé en ce que** la vitesse d'écoulement des particules (20) détectées est estimée à partir du signal de différence.

31. Procédé selon la revendication 30, **caractérisé en ce que** la vitesse d'écoulement des particules (20) détectées est estimée à partir de l'écart temporel entre les valeurs d'extremum du signal de différence.

32. Procédé selon la revendication 31, **caractérisé en ce que** la vitesse d'écoulement des particules (20) détectées est estimée à partir de l'écart temporel entre les maximums des valeurs du signal de différence.

33. Procédé selon les revendications 31 ou 32, **caractérisé en ce que** la répartition des écarts temporels déterminés est définie et subit une analyse statistique.

34. Procédé selon la revendication 33, **caractérisé en ce que** l'analyse statistique des écarts temporels déterminés prend en compte la répartition radiale théorique des vitesses d'écoulement dans un écoulement laminaire.

35. Procédé selon la revendication 34, **caractérisé en ce que** la vitesse d'écoulement du liquide (16) est déterminée à partir du maximum des vitesses estimées des particules en tenant compte de l'écart géométrique entre les bobines réceptrices.

36. Procédé selon la revendication 29, **caractérisé en ce que** l'écart radial estimé entre les particules (20) détectées et la paroi des bobines réceptrices est utilisé pour estimer la vitesse d'écoulement des particules.

37. Procédé selon la revendication 36, **caractérisé en ce que** la dépendance entre la portée d'action des bobines réceptrices (12, 14) vis-à-vis de la distance radiale entre les particules (20) détectées et la paroi des bobines réceptrices est déterminée empiriquement.

38. Procédé selon la revendication 37, **caractérisé en ce que** la répartition radiale théorique des vitesses d'écoulement dans un écoulement laminaire, la distance géométrique entre les bobines réceptrices (12, 14) ainsi que la dépendance déterminée préalablement entre la portée d'action des bobines réceptrices et la distance radiale des particules sont prises en compte dans l'estimation de la distance radiale entre les particules (20) détectées et la paroi des bobines réceptrices.

39. Procédé selon l'une des revendications 26 à 38, **caractérisé en ce que** la taille des particules (20) détectées est estimée à partir du signal de différence et **en ce que** les particules détectées sont classées en fonction de leur taille estimée.

40. Procédé selon la revendication 39 dans la mesure où elle dépend de la revendication 29, **caractérisé en ce que** la distance radiale estimée entre chaque particule (20) détectée et la paroi des bobines réceptrices est utilisée pour estimer la taille des particules.

41. Procédé selon la revendication 40, **caractérisé en ce que** la dépendance entre l'amplitude du signal de différence et la distance radiale entre les particules (20) et la paroi des bobines réceptrices est déterminée empiriquement au préalable.

42. Procédé selon la revendication 41, **caractérisé en ce que** la répartition radiale théorique des vitesses d'écoulement dans un écoulement laminaire, la distance géométrique entre les bobines réceptrices (12, 14), la dépendance déterminée préalablement entre la portée d'action des bobines réceptrices et la distance radiale entre les particules et la paroi des bobines réceptrices ainsi que la dépendance déterminée préalablement entre l'amplitude du signal de différence et la distance radiale des particules par rapport à la paroi des bobines réceptrices sont prises en

20

compte dans l'estimation de la taille des particules (20) détectées.

**43.** Procédé selon l'une des revendications précédentes, **caractérisé en ce qu'**une tension de décalage destinée à compenser une tension erronée de bobine est appliquée sur le signal de sonde avant qu'il soit amené à l'étage (35) activable de conversion A/N.

**44.** Procédé selon la revendication 43, **caractérisé en ce que** la tension de décalage est formée au moyen d'un convertisseur N/A, la valeur de la tension de décalage résultant de la formation de la valeur moyenne du signal numérique de mesure démodulé.

**45.** Procédé selon la revendication 44, **caractérisé en ce que** la formation de la valeur moyenne s'effectue pendant que la tension de décalage est appliquée sur le signal de sonde.

**46.** Procédé selon la revendication 44, **caractérisé en ce que** la formation de la valeur moyenne a lieu avant que la tension de décalage soit appliquée sur le signal de sonde.

Fig. 1

Fig. 2

$A_1 B_1$  $A_2 B_2$  $A_3 B_3$  $A_4 B_4$  $A_5 B_5$  $A_6 B_6$  $A_7 B_7$  $A_8 B_8$  $A_9 B_9$  $A_{10} \frac{B}{10}$

EP 2 028 474 B1

Fig. 3

Fig. 4

Fig. 5

Fig. 6

Fig. 7

Fig. 8

**EP 2 028 474 B1**

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 2108717 A1
- DE 2840358 A1
- KG 47408
- WO 2004081608 A
- WO 2004104561 A
- EP 0778937 A2
- EP 0451209 B1
- DE 3931497 A1
- DE 3117319 A1
- DE 4014756 A1
- US 3575050 A
- DE 2850246 A1
- EP 1189058 A2
- DE 4003330 A1
- US 5175498 A
- US 4445088 A
- WO 2006007826 A1
- EP 0734522 B1